# EUROPEAN PATENT SPECIFICATION

(11) **EP 1 049 487 B1**
(45) Date of publication and mention of the grant of the patent: **22.05.2002**
(21) Application number: 99906067.6
(22) Date of filing: 27.01.1999
(51) Int. Cl.: A61K 38/51, A61K 48/00, C12N 9/88, C12N 15/60

(54) **TREATMENT OF ACUTE INTERMITTENT PORPHYRIA (AIP) AND OTHER PORPHYRIC DISEASES**
BEHANDLUNG VON AKUTER INTERMITTIERENDER PORPHYRIA ODER ANDEREN PORPHYRISCHEN KRANKHEITEN
TRAITEMENT DE LA PORPHYRIE INTERMITTENTE AIGUE (AIP) ET D'AUTRES MALADIES PORPHYRIQUES

(30) Priority: 27.01.1998 DK 11298; 30.12.1998 DK 174898
(43) Date of publication of application: 08.11.2000
(73) Proprietor: Hemebiotech A/S, 3400 Hellerod (DK)
(72) Inventor: FOGH, Jens, DK-3540 Lynge (DK); GELLERFORS, Pär, S-181 63 Lidingö (SE)
(74) Representative: Plougmann, Vingtoft & Partners A/S
(86) International application number: DK9900040
(87) International publication number: WO9937325

(56) References cited:
- GROSS U ET AL: "Haem precursors and porphobilinogen deaminase in erythrocytes and lymphocytes of patients with acute intermittent porphyria." CELLULAR AND MOLECULAR BIOLOGY, (1997 FEB) 43 (1) 29-35, XP002082339
- SASSA S: "Diagnosis and therapy of acute intermittent porphyria." BLOOD REVIEWS, (1996 MAR) 10 (1) 53-8. REF: 24, XP002082340
- GRANDCHAMP B: "Acute intermittent porphyria." SEMINARS IN LIVER DISEASE, (1998) 18 (1) 17-24. REF: 49, XP002082341

## Description

### FIELD OF THE INVENTION

The present invention relates to the use of an effective amount of a catalyst which is an enzyme belonging to the heme biosynthetic pathway for the preparation of a pharmaceutical composition for treating and preventing disease caused by absence or deficiency of the activity of enzymes belonging to the heme biosynthetic pathway. More specifically, the invention pertains to alleviating the symptoms of certain porphyrias, notably acute intermittent porphyria including gene therapy.

### BACKGROUND OF THE INVENTION

### Heme biosynthetic pathway

Heme is a vital molecule for life in all living higher animal species. Heme is involved in such important processes as oxygen transportation (haemoglobin), drug detoxification (cytochrome P450), and electron transfer for the generation of chemical energy (ATP) during oxidative phosphorylation in mitochondria.

Heme is synthesised in eight consecutive enzymatic steps starting with glycin and succinyl-CoA. Sassa S. 1996, Blood Review, 10, 53-58 shows a schematic drawing of the heme biosynthetic pathway indicating that that the first enzymatic step (ALA-synthetase) and the last three steps (coproporphyrinogen oxidase, protoporphyrinogen oxidase and ferrochelatase) are located in the mitochondrion whereas, the remaining are cytosolic enzymes.

Important regulation of the home biosynthetic pathway is delivered by the end product of the metabolic pathway, namely heme, which exerts a negative inhibition on the first rate-limiting enzymatic step (conducted by ALA-synthetase) in the heme biosynthetic pathway, (Strand et al. Proc. Natl. Acad. Sci. 1970, 67, 1315-1320).

Deficiencies in the heme biosynthetic enzymes have been reported leading to a group of diseases collectively called porphyrias.

A defect in the third enzymatic step leads to acute intermittent porphyria, AIP.

### Acute Intermittent Porphyria

Acute intermittent porphyria (AIP) is an autosomal dominant disorder in man caused by a defect (50% reduction of activity) of the third enzyme in the heme biosynthetic pathway, porphobilinogen deaminase, (also known as porphobilinogen ammonialyase(polymerizing)), E.C. 4.3.1.8.(Waldenström J. Acta.Med. Scand. 1937 Suppl.82). In the following, this enzyme will be termed "PBGD".

### Clinical manifestation of AIP

The reduction in enzymatic PBGD activity makes this enzyme the rate limiting step in the heme biosynthetic pathway, with a concomitant increase in urinary and serum levels of delta-aminolevulinic acid (ALA) and porphobilonogen (PBG).

The clinical manifestation of AIP involves abdominal pain and a variety of neuropsychiatric and circulatory dysfunctions. As a result of the enzymatic block, heme precursors such as porphobilinogen (PBG) and delta-aminolevulinic acid (ALA) are excreted in excess amounts in the urine and stool. In acute attacks, high levels of PBG and ALA are also found in serum. These precursors are normally undetectable in serum in healthy individuals.

The neuropsychiatric disturbances observed in these patients are thought to be due to interference of the precursors with the nervous system or due to the lack of heme. For instance, ALA bears a close resemblance to the inhibitory neurotransmitter 4-aminobutyric acid (GABA) and has been suggested to be a neurotoxin. (Jeans J. et al. American J. of Medical Genetics 1996,65, 269-273).

Abdominal pain is the most frequent symptom in AIP patients and occurs in more than 90% during acute attacks, which will be followed rapidly by the development of peripheral neuropathy with weakness in proximal muscles, loss of pinprick sensation, and paraesthesia. Tachycardia, obstipation or diarrhoea may also be present. During acute attacks behavioural changes, confusion, seizures, respiratory paralysis, coma and hallucinations may be present.

Hypertension is also associated with AIP, with as high as 40% of patients showing sustained hypertension between attacks. An association between chronic renal failure (Yeung L. et al. 1983, Q J. Med 52, 92-98) and AIP as well as hepatocellular carcinoma. (Lithner F. et al. 1984, Acta.Med.Scand. 215,271-274), has been reported.

The AIP is a lifelong disease, which usually becomes manifest in puberty.

### Factors precipitating acute attacks.

Most precipitating factors exhibit an association with the first rate-limiting enzyme in the heme biosynthetic pathway through heme, the final product of the pathway. A lowering of the heme concentration will immediately increase the rate of ALA-synthetase. An overproduction of ALA then makes the partially deficient PBGD enzyme (50% activity) now rate-limiting with an accumulation of the heme precursors ALA and PBG. Drugs that induces cytochrome P450 such as barbiturates, estrogens, sulphonamides, progesterone, carbamyazepine, and phenytoin can all precipitate acute attacks. (Wetterberg L. 1976, In Doss M. Nowrocki P. eds. Porphyrias in Human Disease. Reports of the discussion. Matgurg an der Lahn, 191-202).

The clinical manifestation is more common in women, especially at time of menstruation. Endocrine factors such as synthetic estrogens and progesterone are known precipitating factors. A significant factor is also the lack of sufficient caloric intake. Hence, caloric supplementation during acute attacks reduces clinical symptoms. (Wetland F.H. et al. 1964, Metabolism,13, 232).

Finally, various forms of stress including illness, infections, surgery and alcoholic excess have been shown to lead to precipitation of acute attacks. There are also cases of acute attacks where no precipitating factor can be identified.

### Prevalence of AIP

Prevalence of 0.21% has been reported (Tishler P.V: et al. 1985, Am.J.Psychiatry 142,1430-1436), with as high a prevalence as 1 per 1500 in geographic isolates in northern Sweden (Wetterberg L. 1967, Svenska bokförlaget Nordstedt,Stockholm). Prevalence up to 200 per 10,000 inhabitants have been reported from Arjepong in Northern Sweden. (Andersson, Christer, Thesis, 1997, ISBN 91/7191/280/0, pp. 22-23).

### Existing treatment of AIP

The treatment of AlP as well as of other types of porphyrias such as variegata, hereditary coproporphyria, harderoporphyria, and aminolevulinic acid dehydratase deficiency, are basically the same. Existing therapies for AIP, are all aimed at reducing circulating PBG and ALA by inhibiting the first rate-limiting enzymatic step ALA-synthetase. This inhibition of ALA-synthetase is achieved by increasing circulating heme, since heme is a negative feed back regulator of ALA-synthetase. Hematin treatment, high caloric intake or inhibition of heme breakdown by Sn-mesoporphyrin administration are the existing therapies today. These therapies have shown limited efficacy.

Treatment between acute attacks involves sufficient caloric intake and avoidance of drugs and immediate treatment of infections.

Patients that experience acute attacks are treated with intravenous carbohydrates usually dextrose (300 g/day) and intravenous hematin (3 - 8 mg/(kg day)).

Treatments with long acting agonistic analogues of LHRH, have been shown to reduce the incidence of pre-menstrual attacks by inhibiting ovulation in AIP patients. Finally, treatments involving heme analogues Sn-mesoporphyrin, which inhibit heme breakdown have also been attempted.

### Medical need in AIP

The lack of effective treatment for AIP is well recognised. In a US mortality study in AIP patients requiring hospitalisation it was concluded that the mortality rate was 3.2-fold higher as compared to a matched general population.

Suicide was also a major cause of death, occurring at a rate of 370 times that expected in the general population (Jeans J. et al. 1996, Am. J. of Medical Genetics 65, 269-273).

Hematin therapy is usually initiated when high caloric intake is not sufficient to alleviate acute attacks. Studies with hematin have been performed but these studies generally used the patients as their own control after the patients did not respond to high carbohydrate treatment. (Mustajoki et al. 1989, Sem. Hematol. 26, 1-9).

The one controlled study with hematin treatment reported, failed to reach statistical significance due to too small a patient number (Herrick A.L. et al 1989, Lancet 1, 1295-1297).

In conclusion, there is a definite need for the provision of novel therapeutic/prophylactic methods aimed at these diseases.

### DISCLOSURE OF THE INVENTION

Levels of ALA and PBG found in urine in patients with symptomatic AIP, are in the range of 1-203 mg/day and 4-782 mg/day, respectively. Normal excretion of ALA and PBG is very low (0-4 mg/day). Important is the observation that these patients also have elevated levels of ALA and PBG in serum. It was shown in a study that AIP patients had significantly elevated levels of ALA (96 µg %) and PBG (334 µg %) in serum in connection with acute attacks and that the severity of the attacks were correlated to high levels of ALA and PBG. Hence, it is important to reduce the circulating levels of ALA and PBG in order to eliminate clinical symptoms and to normalize the heme pool.

The present inventors present a new therapeutic rational in the treatment of AIP, a rationale using PBGD, preferably recombinant PBGD (rPBGD) in order to reduce circulating high levels of PBG in serum by metabolising (by enzymatic conversion) PBG to hydroxymethylbilane (HMB), which is the normal product of the reaction. This substitution therapy will lead to a normalization of PBG in serum as well as to a normalization of the heme pool. It will also lead to a normalization of ALA in serum, since these heme precursors are in equilibrium with each other. A lowering of serum ALA and PBG is expected to result in a concomitant relief of symptoms. The product of the reaction (HMB) will diffuse back into the cells and enter the normal heme biosynthetic pathway and will become subsequently metabolized to heme.

Hence, PBGD administered by injections will carry out its normal catalytic function by converting PBG to HMB in serum (extracellularly, not inside the cells), where it normally functions. The new therapeutic idea is based on the assumption that ALA, PBG and HMB permeate cellular membranes or are transported specifically across them. An alternative to this is to administer a form of PBGD which will be able to act intracellularly, either as a consequence of formulation or as consequence of modification of PBGD so as to facilitate its entry into cells from the extracellular compartment.

The observation that AIP patients have large amounts of these heme precursors in the serum supports the idea that PBG does not accumulate intracellularly, but is released from the cells into serum when the intracellular concentration increases due to the PBGD enzymatic block.

The basic new therapeutic concept for AIP is valid for all porphyrias and therefore the invention is in general aimed at treating these diseases by substituting the reduced or missing enzymatic activity characterizing the porphyrias.

Hence, in its broadest aspect, the invention pertains to the use of an effective amount of a catalyst which is an enzyme belonging to the heme biosynthetic pathway or an enzymatically equivalent part or analogue thereof together with a pharmaceutically acceptable carrier for the preparation of a pharmaceutical composition for treatment or prophylasis of a disease caused by a defiency, in a subject, of an enzyme belonging to the heme biosynthetic pathway.

Alternatively investigations in treating the porphyrias have also suggested gene therapy, thus aiming at introducing genetic material in relevant cells, which will then take over the *in vivo* production of the enzyme of interest.

Hence, by the term "catalyst" is herein meant either the relevant enzyme which is substituted as it is, or an enzymatically equivalent part or analogue thereof. One example of an enzymatically equivalent part of the enzyme could be a domain or subsequence of the enzyme which includes the necessary catalytic site to enable the domain or subsequence to exert substantially the same enzymatic activity as the full-length enzyme or alternatively a gene coding for the catalyst.

An example of an enzymatically equivalent analogue of the enzyme could be a fusion protein which includes the catalytic site of the enzyme in a functional form, but it can also be a homologous variant of the enzyme derived from another species. Also, completely synthetic molecules which mimic the specific enzymatic activity of the relevant enzyme would also constitute "enzymatic equivalent analogues".

In essence, the inventive concept is based on the novel idea of substituting the reduced enzymatic activity in the subject simply by administering a catalyst which will "assist" the enzyme which is in deficit. The precise nature, however, of the catalyst is not all-important. What is important is merely that the catalyst can mimic the enzymatic *in vivo* activity of the enzyme.

The term "the heme biosynthetic pathway" refers to the well-known enzymatic steps (cf. e.g. Sassa S. 1996, Blood Review, 10, 53-58) which leads from glycin and succinyl-CoA to heme, and enzymes belonging to this synthetic pathway are porphobilinogen deaminase (PBGD), ALA dehydratase, Uroporphyrinogendecarboxylase, Coproporphyrinogen oxidase, Coproporphyrinogen oxidise, Protoporphyrinogen oxidase, Uroporphyrinogen III synthase, Ferrochelatase, and Uroporphyrinogen decarboxylase. Hence, in line with the above, a catalyst used according to the invention is such an enzyme or an enzymatically equivalent part or analogue thereof. It should be noted that all of the above-mentioned enzymes have been sequenced, thus allowing recombinant or synthetic production thereof.

The diseases related to reduced activity of these enzymes are acute intermittent porphyria (AIP), ALA deficiency porphyria (ADP), Porphyria cutanea tarda (PCT), Hereditary coproporphyria (HCP), Harderoporphyria (HDP), Variegata porphyria (VP), Congenital erythropoetic porphyria (CEP), Erythropoietic protoporphyria (EPP), and Hepatoerythropoietic porphyria (HEP).

By the term "effective amount" is herein meant a dosage of the catalyst which will supplement the lack or deficiency of enzymatic activity in a subject suffering from porphyria caused by reduced activity of one of the above-mentioned enzymes. The precise dosage constituting an effective amount will depend on a number of factors such as serum half-life of the catalyst, specific activity of the catalyst etc. but the skilled person will be able to determine the correct dosage in a given case by means of standard methods (for instance starting out with experiments in a suitable animal model such as with transgenic animals so as to determine the correlation between blood concentration and enzymatic activity).

The disease which is the preferred target for the inventive method is AIP, and therefore the catalyst is PBGD or an enzymatically equivalent part or analogue thereof. It is most preferred that the catalyst is a recombinant form of the enzyme belonging to the heme biosynthetic pathway or of the enzymatically equivalent part or analogue thereof, since recombinant production will allow large-scale production which, with the present means available, does not seem feasible if the enzyme would have to be purified from a native source.

Preferred formulations and dosage forms of the catalyst are exemplified for, but not limited to, PBGD in the detailed description hereinafter, and these formulations also are apparent from the claims. It will be appreciated that these formulations and dosage forms are applicable for all catalysts used according to the invention.

One important embodiment of the invention is one wherein the catalyst, upon administration, exerts at least part of its enzymatic activity in the intracellular compartment. This can e.g. be achieved when the catalyst is an enzymatically equivalent part or analogue of the enzyme, since such variations of the enzyme can be tailored to render them permeate cell membranes. Hence, when the catalyst is a small artificial enzyme or an organic catalyst which can polymerize porphobilinogen to hydroxymethylbilane, it should be possible for the skilled man to introduce relevant side chains which facilitates entry into the intracellular compartment. Alternatively, the catalyst is the enzyme, but formulated in such a manner that it exerts at least part of its enzymatic activity intracellularly upon administration to the subject. This can be achieved by tagging the enzyme with specific carbohydrates or other liver cell specific structures for specific liver uptake, *i.e.* the enzyme (or analogue) is modified so as to facilitate active transport into e.g. liver cells.

Although the above embodiments are interesting, it is believed that the normal, practical embodiment of the invention will involve use of a catalyst which exerts substantially all its enzymatic activity extracellularly in the bloodstream, since it is believed that the metabolic products of the enzymatic conversion of the relevant heme precursor will permeate freely into the intracellular compartment where the remaining conversions of the heme biosynthetic pathway can take place. Alternatively, the metabolic product may be excreted from the subject via urine and/or faeces at least to some extent.

As mentioned above, it is preferred that the catalyst is produced recombinantly, *i.e.* by a method comprising
a) introducing, into a suitable vector, a nucleic acid fragment which includes a nucleic acid sequence encoding the catalyst;
b) transforming a compatible host cell with the vector;
c) culturing the transformed host cell under conditions facilitating expression of the nucleic acid sequence; and
d) recovering the expression product from the culture
and optionally subjecting the expression product to post-translational processing, such as in vitro protein refolding, enzymatic removal of fusion partners, alkylation of amino acid residues, and deglycosylation, so as to obtain the catalyst.

For relatively small catalysts (e.g. those constituted mainly of the active site of the enzyme), the catalyst can alternatively be prepared by liquid-phase or solid-phase peptide synthesis.

A more detailed explanation of the recombinant production of the model enzyme PBGD is given in the detailed section hereinafter, but as mentioned herein the same considerations apply for all other peptide catalysts of the invention. One of the main advantages of producing the catalyst by recombinant or synthetic means is, that if produced in a non-human cell, the catalyst is free from any other biological material of human origin, thus reducing problems with known or unknown pathogens such as viruses etc.

The dosage regimen will normally be comprised of at least one daily dose of the catalyst, (preferably by the intravenous route). Normally 2, 3, 4 or 5 daily dosages will be necessary, but if sustained release compositions are employed, less than 1 daily dosage are anticipated.

The daily dosage should be determined on a case by case basis by the skilled practitioner, but as a general rule, the daily dosage will be in the range between 0.01 - 1.0 mg/kg body weight per day of the catalyst. More often the dosage will be in the range of 0.05 - 0.5 mg/kg body weight per day, but it should never be forgotten that precise dosage depends on the dosage form and on the activity of the catalyst as well as on the degree of deficiency of the relevant enzyme and an individualized treatment, where the dose is adjusted to normalize patient serum and urine precusor levels.

The most correct way of determining the correct dosage is based on the patient specific precursor levels. The precursor being the product of the enzymatic reaction.

For PBGD, the daily dosage is about 0.08-0.2 mg per kg body weight per day, and most often 0.1 mg per kg body weight per day will be the dosage of choice. It is believed that comparable dosages will be applicable for the other full-length enzymes.

Finally, as will be appreciated from the above disclosure, the invention is based on the novel idea of providing substitution for the enzymes lacking in activity. To the best of the knowledge of the inventors, therapeutic use of catalysts having such effects have never been suggested before, and therefore the invention also pertains to a catalyst as defined herein for use as a pharmaceutical. Furthermore, use of such catalysts for the preparation of pharmaceutical compositions for treatment of the above-discussed diseases is also part of the invention.

Legends to figures:
Figure 1: Cicular map of PBGD clone # 1.1 in pBluescript SK
Figure 2: Circular map of expressed plasmid pExp0
Figure 3: Circular map of expressed plasmid pPBGD 1.1
Figure 4: Circular map of expressed plasmid pKK223-3
Figure 5: Circular map of expressed plasmid pExp1
Figure 6: Circular map of expressed plasmid pPBGD 1.1 Tra
Figure 7: Circular map of expressed plasmid pExp1-M2
Figure 8: Circular map of expressed plasmid pExp1-M2-Puc-BB
Figure 9a-9x: PBGD clone # 1.1 in pBluescript SK - Sequence

### DETAILED DISCLOSURE OF THE INVENTION

In a first embodiment the invention relates to the use of an effective amount of a catalyst which is an enzyme belonging to the heme biosynthetic pathway or an enzymatically equivalent part or analogue thereof together with a pharmaceutically acceptable carrier for the preparation of a pharmaceutical composition for treatment or prophylaxis of a disease caused by a deficiency, in a subject, or an enzyme belonging to the heme biosynthetic pathway. The disease may be selected from the phorphyria group and the catalyst may be an enzyme selected from the group consisting of porphobilinogen deaminase (PBGD)
ALA dehydratase,
Uroporphyrinogendecarboxylase,
Coproporphyrinogen oxidase,
Coproporphyrinogen oxidise,
Protoporphyrinogen oxidase,
Uroporphyrinogen III synthase,
Ferrochelatase, and
Uroporphyrinogen decarboxylase,
or an enzymatically equivalent part or analogue thereof.

In a preferred embodiment, the disease is AIP and the enzyme is PBGD or an enzymatically equivalent part or analogue thereof. In a further embilment, the catalyst is a recombinant form of the enzyme belonging to the home biosynthetic pathway or of the enzymatically equivalent part or analogue thereof.

The catalyst may be administrable by a route selected from the group consisting of the intravenous route, the intraarterial route, the intracutaneous route, the subcutaneous route, the oral route, the buccal route, the intramuscular route, the anal route, the transdermic route, the intraderrnal route, and the intratechal route.

The catalyst is preferrable formulated in an isotonic solution, such as 0.9% NaCl and 10-50 mM Sodium phosphate pH 7.0 +/- 0.5 up to pH 8.0 or Sodium phosphate, glycine, mannitol or the corresponding potassium salts. The catalyst may also be lyophilised, sterile filtered, and in a further embodyment formulated as lipid vesicles comprising phosphatidylcholine or phosphatidylethanolamine or combinations thereof. In a still other embodiment the catalyst is incorporated into erythrocyte ghosts.

Also a sustained release formulation may be perforrmed involving biodegradable microspheres, such as microspheres comprising polylactic acid, polyglycolic acid or mixtures of these.

A further method according to the invention is wherein the catalyst is lyophilized in a two-compartment cartridge, where the catalyst will be in the front compartment and water for reconstitution in the rear compartment. The two compartment cartridge may be combined with an injection device to administer the catalyst either by a needle or by a needle-less (high pressure) device.

It may also be very convenient to administer the catalyst in a formulation of a physiological buffer containing an enhancer for nasal administration.

Other formulations for the catalyst include an oral formulation containing lipid vesicles, such as those comprising phospatidylcholine, phosphatidylethanolamine, or sphingomyeline, or dextrane microspheres.

The formulation is preferable one which is able to enhance the half-life of the catalyst in the subject's bloodstream. This may by use of a formulation wherein the catalyst has a polyethylene glycol coating.

The catalyst may also be complexed with a heavy metal.

In a further aspect the catalyst is an enzymatically equivalent part or analogue of the enzyme and exerts at least part of its enzymatic activity intracellularly upon administration to the subject.This may be when the catalyst is a small artificial enzyme or an organic catalyst which can polymerize porphobilinogen to hydroxymethylbilane.

Furthermore, the catalyst may be said enzyme formulated in such a manner that it exerts at least part of its enzymatic activity intracellularly upon administration to the subject.

In addition the catalyst may be tagged with specific carbohydrates or other liver cell specific structures for specific liver uptake.

In a furhter aspect the catalyst exerts substantially all its enzymatic activity extracellularly in the bloodstream.

In a still further aspect, the enzymatic activity of the catalyst on its relevant heme precursor results in a metabolic product which 1) either moves into the intracellular compartment and is converted further via the remaining steps of the heme biosynthetic pathway or 2) is excreted from the subject via urine and/or faeces.

A furhter embodiment of the invention relates to a use wherein the catalyst has been prepared by a method comprising
a) introducing, into a suitable vector, a nucleic acid fragment which includes a nucleic acid sequence encoding the catalyst;
b) transforming a compatible host cell with the vector;
c) culturing the transformed host cell under conditions-facilitating expression of the nucleic acid sequence; and
d) recovering the expression product from the culture
and optionally subjecting the expression product to post-translational processing, such as in vitro protein refolding, enzymatic removal of fusion partners, alkylation of amino acid residues, and deglycosylation, so as to obtain the catalyst.

The catalyst may be prepared by liquid-phase or solid-phase peptide synthesis and it is preferable free from any other biological material of human origin.

As mentioned above the catalyst may be administrable at least once a day, such as 2, 3, 4, and 5 times daily depending on the specific treatment regimen outlined for the patient in that precursor levels for each patient are measured before and/or during treament for evaluation of the specific dosage.

Accordingly the daily dosage may be in the range of 0.01 - 1.0 mg/kg body weight per day, such as in the range of 0.05 - 0.5 mg/kg body weight per day. And the present invention also relates to the use of the catalyst for the preparation of a pharmaceutical composition.

It is estimated that a dosage will often be about 0.1 mg per kg body weight per day.

Accordingly, the invention also relates to a catalyst which is an enzyme of the heme biosynthetic pathway or an enzymatically equivalent part or analogue thereof, for use as a medicament. Thus in a further embodyment, the invention relates to a catalyst which is an enzyme of the heme biosynthetic pathway or an enzymatically equivalent part or analogue thereof for the preparation of a pharmaceutical composition for the treatment or prophylaxis of diseases caused by deficiency of said enzyme.

Naturally, the catalyst may be a recombinant form of the enzyme. An example is a recombinant human PBCD based on any of Seq. ID NO 1 (clone PBGD 1.1) and Seq. ID NO 12 (non-erythro PBGD 1.1.1).

In a preferred embodiment and as will be disclosed in detail below, the invention also relates to the use of a human PBGD cDNA sequence of either non-Erythropoietic form or Erythropoietic form for the preparation of a medicamant for gene therapy by transfection of the patient with the human PBGD cDNA sequence of either non- Erythropoietic form or Erythropoletic form according to the tissue in which PBGD should be expressed, for treatment or preventing disearse in a patient having a mutation in the PBGD gene causing an enzyme defect. Preferably the enzyme defiency is selected from enzyme defiencies resulting in a disease selected from Acute Intermittent Porphyria, (AIP), ALA deficiency porphyria (ADP), Porphyria cutanea tarda (PCT), Hereditary coproporphyria (HCP), Harderoporphyria (HDP), Variegata porphyria (VP), Congenital erythropoietic porphyria (CEP), Erythropoietic protoporphyria (EPP), and Hepatoerythropoietic porphyria (HEP).

In a preferred embodiment, the human PBGD cDNA sequence is selected from Seq. ID NO 1 (clone PBGD 1.1) and Seq. ID NO 12 (non-erythro PBGD 1.1.1).

The transfection may be by use of a vector selected from adenovirus, retrovirus and associated adenovirus. The PBGD gene transfer vector into human cells (erythropoeitic and/or non-erythropoietic) preferably results in normal PBGD activity or in an activity wherin the patient is free of symptoms of disease.

The use of a specific designed oligonucleotide molucule for the manufacture of a medicament for the gene therapy treatment of patients with Acute Intermittent Porphyria (AIP) by a correction of one of the specific point mutations identified causing AIP by use of chimeraplasty gene repair is also envisaged. involves specific designed oligonucleotides and a specific knowledge of both the mutation to be corrected and the sequence on both sides on the mutation.

In a specific embodiment of chimeraplasty gene repair is by use of a delivery system for transfection by use of non-viral vectors formulated in a vehicle preparation comprising one or more components selected from cationic phospholipids, phospholipids, phospholipids mixed with neutral lipids, lictosylated PEI, liposomes comprising mixtures of natural phopholipids and neutral lipids.

The mutation may be selected from the mutations shown in Table A.

The following description of preferred embodiments of the invention will focus on recombinant production of PBGD and formulations and uses thereof. It will be appreciated, however, that all disclosures relating to this polypeptide apply also for the other enzymes mentioned above. Hence, production and use of PBGD only exemplifies the invention, but all other enzymes of the heme biosynthetic pathway can substitute PBGD in the embodiments described hereinafter.

### Production of recombinant PBGD

As mentioned above, it is preferred to administer recombinant versions of the various enzymes of the heme biosynthetic pathway. In the following will be described recombinant production of one of these enzymes, namely PBGD.

The gene for the erythropoietic PBGD, which is located in the human genome in the chromosomal region 11q 24, is composed of 15 exons spanning 10 kb of DNA and is shown in Grandchamp B. et al. 1996. J. of Gastroenerology and Hepatology 11. 1046-1052.

The gene coding the erythropoietic PBGD enzyme (344 amino acids) (Raich N. et al 1986, Nucleic. Acid. Res, 14, 5955-5968), will be cloned from a human erythropoietic tissue by reverse transcriptase or amplification by PCR (polymeraso chain reaction) of the PBGD coding region.

The gene will be inserted in a plasmid and transformed into a suitable host cell (a bacterium such as E. coli and B. subtilis, a fungus such as Saccharomyces, or a mammalian cell line, such as CHO cells). The expression of the PBGD gene will be regulated by a promoter which is compatible with the selected host cell.

For bacterial production: An endogenous ATG sequence is located at the NH2-terminal end of the rPBGD structural gene for initiation of translation and cytoplasmic expression. Alternatively insert in front of the PBGD coding region a bacterial signal sequence for example an E. coli periplasmic enzyme signal peptide or a signal peptide from a secreted enterotoxin or endotoxin in E. coli, to obtain secretion in E. coli.

The plasmid used for production of rPBGD in E. coli was constructed in the following way:

### Construction of the rPBGD production plasmid

### Introduction:

The erythropoietic expressed form of porphobilinogen deaminase (PBGD) ( Raich N, et al Molecular cloning and complete primary sequence of erythrocyte porphobilinogen deaminase. Nucleic Acids Research 1986 14(15): 5955-67) was cloned and sequence determined. Two forms of PBGD are known. The erythropoietic form is expressed specifically in erythroid progenitors and the constitutive form is expressed in all cells (Grandchamp B, et al Tissue-specific expression of porphobilinogen deaminase. Two isoenzymes from a single gene. Eur J Biochem. 1987 Jan; 162(1):105-10. mp et. al. 1987.) The two are expressed from the same gene and are identical except for the addition of 17 amino acids at the amino terminus of the constitutive form through alternative exon usage. It was decided to clone and express the erythropoietic form. There are three sequences for PBGD in the Genebank, the two isoforms mentioned above and the genomic sequence (Yoo HW, et al Hydroxymethylbilane synthase: complete genomic sequence and amplifiable polymorphisms in the human gene. Genomics 1993 Jan; 15(1):21-9). These all have nucleotide differences translating to amino acid changes. Before choosing a specific sequence to be expressed for a human therapeutic it was therefore necessary to determine what is the wild type allele. To accomplish this, PBGD cDNA clones were isolated and sequenced from a number of sources to define the most common amino acid usage. Oligonucleotide primers were designed to amplify the coding region from cDNAs by Polymerase Chain Reaction (PCR) (Saiki RK, et al. Enzymatic amplification of beta-globin genomic sequences and restriction site analysis for diagnosis of sickle cell anemia. Science 1985, Dec 20; 230(4732):1350-4). These were used to isolate cDNAs from 5 sources of mRNA which were then cloned into a plasmid vector. Eight of these clones were sequenced and along with the published sequences define a wild type allele, which should be the most common amino acid sequence in the population. This wild type allele will be used for protein expression.

### Strategy:

A nested PCR strategy was devised to clone PBGD. The first primer set, (see Table 1) Ico379 and Ico382, are 20mers that bind to sequence outside of the coding region. Ico379 is specific for the 5' untranslated region of the mRNA (cDNA) of the erythropoietic form of PBGD. The binding site is in an exon region not expressed in the constitutive form of the enzyme. Ico382 binds to the 3' untranslated region of both forms of PBGD. Internal to these are a second set of oligonucleotide primers to be used for the second round of PCR, Ico375 and Ico376, designed to distal ends of the PBGD coding region. Ico375 has 22 bases of sequence homologous to the 5' end of the coding region of the erythropoietic form of PBGD with the ATG start codon followed by an EcoRI endonuclease cleavage site for cloning of the PCR product and 4 bases of sequence to ensure efficient restriction. Ico376 has 33 bases homologous to the 3' end of the PBGD coding region with 3 bases changed internally to introduce a Munl/Mfel endonuclease cleavage site through silent mutations and ending with the TAA stop codon. This restriction site will be used to easily introduce sequence encoding a His-Tag to the DNA with oligonucleotide adapters or to enable other 3' modifications. Following the stop codon is a second stop codon to ensure good termination of translation and a HindIII endonuclease cleavage site for cloning the PCR product followed by 4 bases to ensure efficient restriction. The EcoRI and HindIII endonuclease cleavage sites introduced onto the ends of the PBGD PCR product ligate into the respective unique restriction sites in the high copy number pBluescriptll SK-(Stratagene) vector for sequencing and will then be used to move the PBGD DNA into an *E. coli* expression vector for production of recombinant human porphobilinogen deaminase, rh-PBGD.

### PCR:

Six cDNAs were used as a PCR source; spleen, bone marrow, lymph node, lung, whole brain and adipose tissue each from a different pool of human donors (produced by Donald Rao using BRL Superscript II with 500 ng Clontech poly-A RNA in 20 µl reaction volumes per manufacturers instructions except adipose which was made from 5 µg of Clontech total RNA from a single donor). List of equipment and supplies used (see lists below). One µl of each cDNA (approximately 25ng) was amplified with Advantage cDNA polymerase mix (Clontech) with 0.2mM dNTP (PE/ABI) and 0.3µM each of lco379 and lco382 in 50 µl reaction volumes. Two cycle PCR was used, with an initial heat denaturation step at 94°C for 1' 40" then 28 cycles of 96°C for 16" and 68°C for 2'. A final extension of 6' at 74°C ensured that extension products were filled out. One fifth of the reaction was run out on a 1.2% agarose gel with 2 µl of 6X ficol loading dye in 0.5X TBE buffer (Maniatis T., E.F. Fritsch, J. Sambrook. Molecular Cloning (A laboratory Manual) Cold Spring Harbor Laboratory. 1982 ). The predicted band of 1.1 kb. was observed by ethidium bromide staining with all sources but lung tissue cDNA. These bands were excised and DNA was isolated with Microcon-30 with micropure inserts (Amicon/Millipore) per manufacturers instructions and buffer exchanged with dH20. One tenth of the recovered DNA was amplified with Advantage cDNA polymerase mix (Clontech) with 0.2mM dNTP (PE/ABI) and 0.3µM each of the internal nested oligonucleotides (lco375 and lco376) at 0.3µM in 50µl reactions. Two cycle PCR was used again with an initial heat denaturation step at 94°C for 1' 40" then 2 cycles of 96°C for 16" and 68°C for 2' then 13 cycles of 96°C for 16" and 72°C for 2' with a final extension of 6' at 74°C. Ten µl of the 50 µl reactions were run on a 1.2% agarose gel with 2 µl 6X loading dye. The resulting bands were of the expected size, 1.05 kb. The remainder of the PCR reactions were passed through Chromaspin-400 columns (Clontech) per manufacturers instructions to remove reaction components and to exchange buffer with TE (10mM Tris-HCI pH8.0/1mM EDTA). The DNA containing eluates were washed with dH20 and concentrated with Microcon-100 spin-filters (Amicon/Millipore) per manufacturers instructions.

### Cloning:

The purified PBGD DNA was digested for 6 hours with 40 Units each of EcoRI and HindIII in EcoRI "U" buffer (New England Biolabs (NEB)) in 50 µl reactions at 37°C. Enzymes were heat killed for 20 minutes at 68°C and reactions were spun in Microcon 100 spin-filters to remove small DNA end pieces, washed with dH2O and concentrated. One fifth of the resulting DNA was ligated with approximately 50 ng EcoRI and HindIII digested and twice gel purified pBluescriptII SK- (Stratagene) and 200 units T4 DNA ligase (NEB cohesive end units) for 15 hours at 16°C. The ligase was heat killed at 75°C for 10 minutes. The reactions were then buffer exchanged with dH20 and concentrated in Microcon-100 spin filters and volumes taken up to 5 µl with dH2O. One µl each was electroporated into 25 µl DH10B Electromax cells (Gibco/BRL) at 2.5Kv/2000hms/25µF in 0.1 cm cuvets with a BioRad electroporator. One ml of SOC medium (Gibco/BRL) was added and the cells were outgrown at 37°C for one hour at 250 rpm. Cells were plated out on LB plates (Maniatis T., E.F. Fritsch, J. Sambrook. Molecular Cloning (A laboratory Manual) Cold Spring Harbor Laboratory. 1982) with 150 µg/ml ampicillin. The efficiency of all five were approximately twice background (vector ligated without insert.) Colony PCR was used to analyze 18 transformants of each electroporation for the presence of PBGD. An internal PBGD specific primer (lco381) was used with a pBluescript specific primer (lco385) to both confirm identity and proper orientation in the vector. The 25 µl reactions were set up on ice to inactivate proteases with primer concentrations of 0.4µM, 0.125U Taq polymerase (Fisher), and 0.2mM dNTP(PE/ABI.) Two cycle PCR was used, with an initial heat denaturation step at 94°C for 1' 40" a further denaturing step at 96°C for 20 seconds, then 30 cycles of 96°C for 16" and 68°C for 1' with a final extension of 4' at 74°C. Five µl of 6X loading dye was added and 12.5 µl each were run out on a 1.2% agarose gel. Results are as follows: 12/18 positive colonies for spleen; 10/18 for bone marrow; 8/18 for lymph node; 9/18 for brain and 10/18 for adipose tissue. Two positive colonies each for the first 3 and 1 each for the latter two were grown up in 25 ml. liquid LB culture with 150 µg/ml ampicillin over night at 37°C with 250 rpm. Plasmid DNA was purified from the cultures with Qiagen's Tip-100 DNA purification kit per manufacturer's instructions. UV absorbance at 260nm was used to determine the plasmid yields which varied from between 131 and 169 µg of highly purified DNA.

### Sequencing:

Sequencing reactions of double stranded plasmid DNA with Big Dye terminator cycle sequencing were performed in a 9700 thermocycler (Perkin Elmer/Applied Biosystems.) Two vector primers (Ico383 and Ico385) and two PBGD specific internal primers (Ico380 and Ico381) were used for all 8 plasmids (see sequence). In addition a fifth vector primer (Ico285) was used for the brain and adipose derived clones. Reaction conditions were per manufacturers protocol as follows: 500 ng plasmid DNA and 4 pmol oligonucleotide primer with 8 µl ready mix in 20 µl volumes with 30 cycles of 96°C for 12" and 60°C for 4'. Extension products were purified by isopropanol precipitation. To each reaction 20 µl of dH2O and 60 µl Isopropanol were added. These were mixed by inversion and allowed to sit at room temperature for 15 minutes then spun for 40' at 3250 rpm in a Beckman GS-6KR centrifuge with the GH3 rotor and Microplate + carriers. Reactions were inverted then spun at 1680 rpm for 1' to remove liquid from the pelleted DNA. DNA sequence analysis was performed at the University of Washington Biochemistry Department sequencing Laboratory with an Applied Biosystems 377 sequencer.

### Analysis:

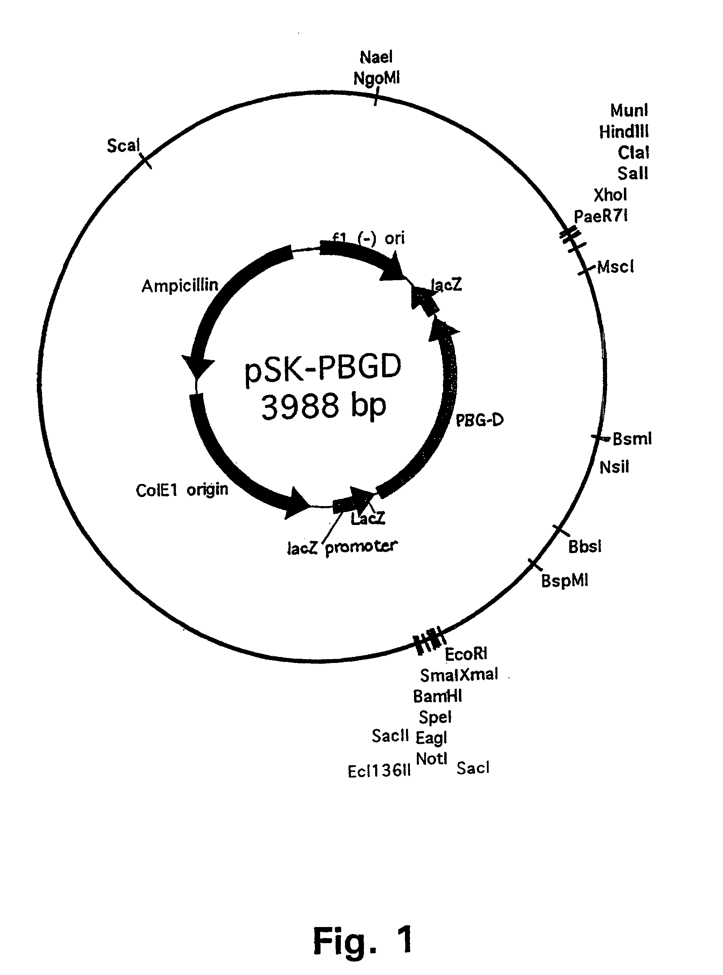

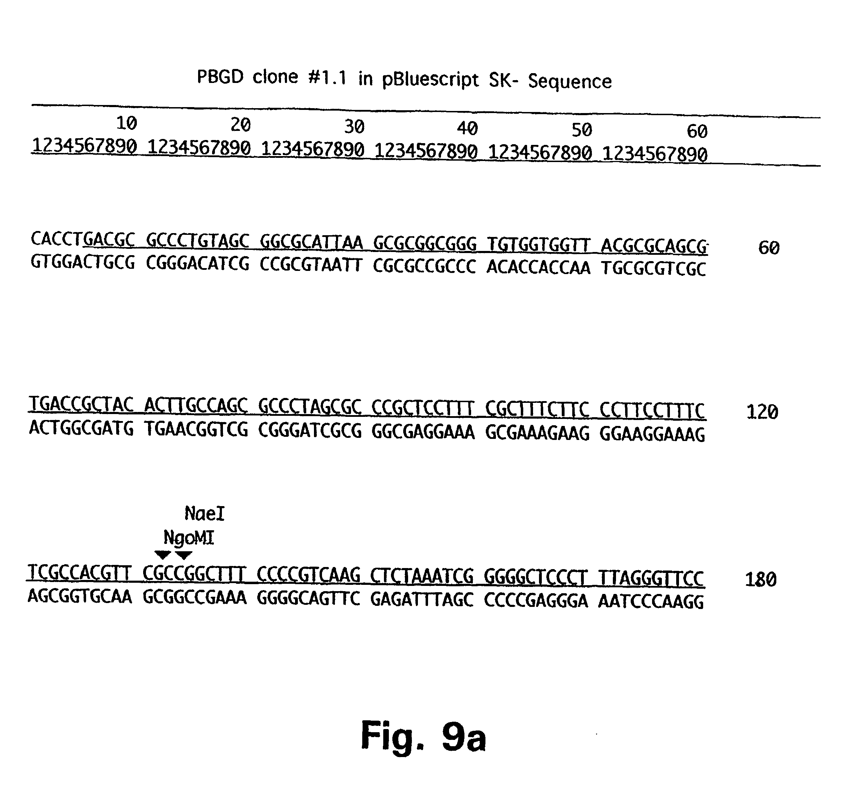

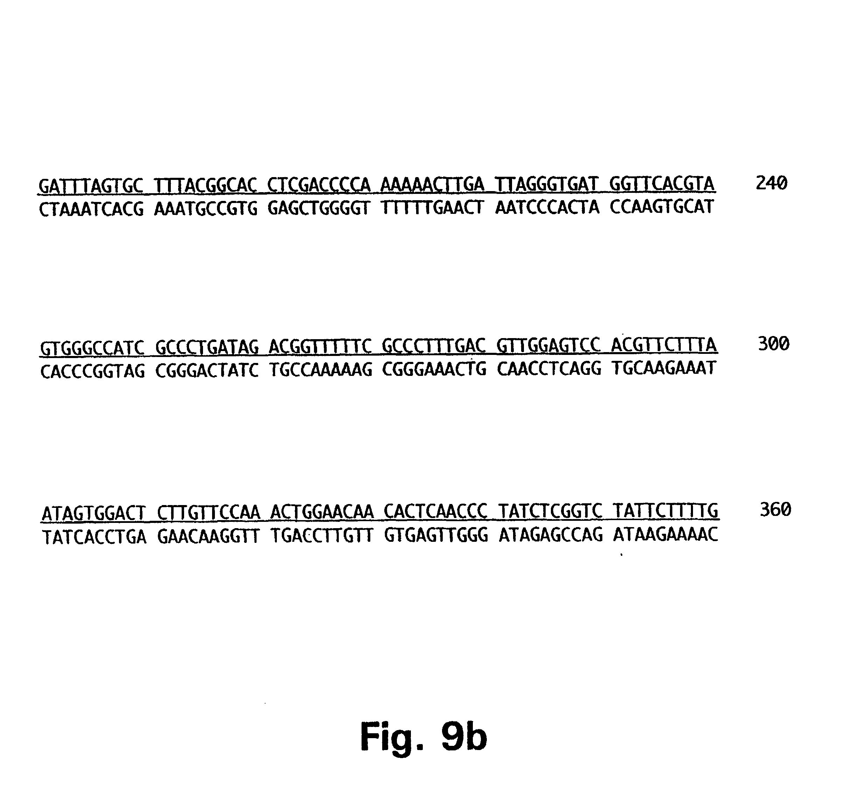

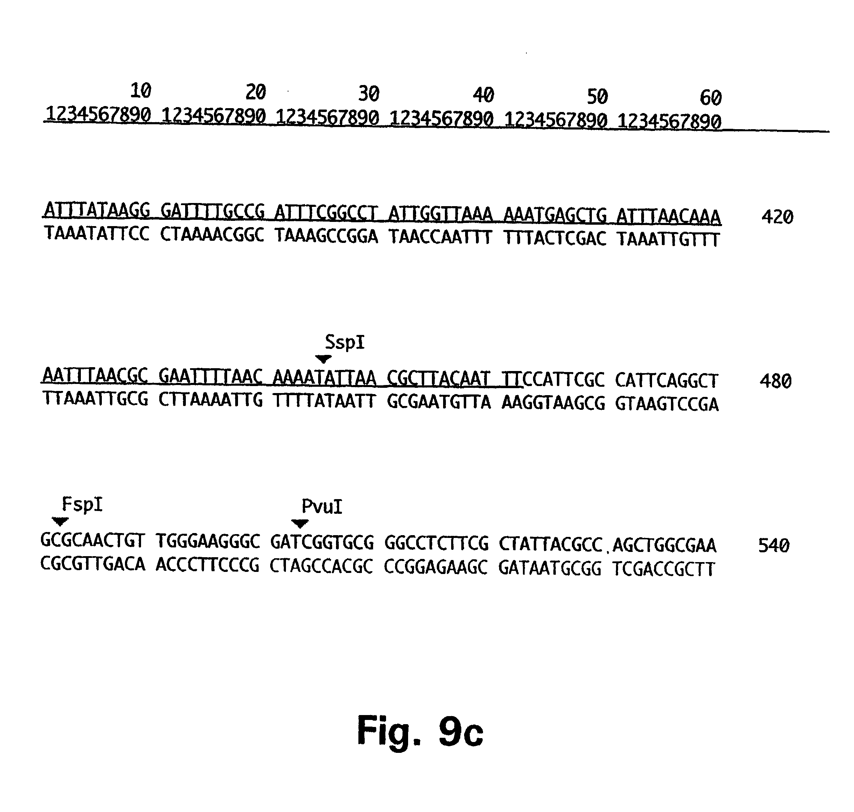

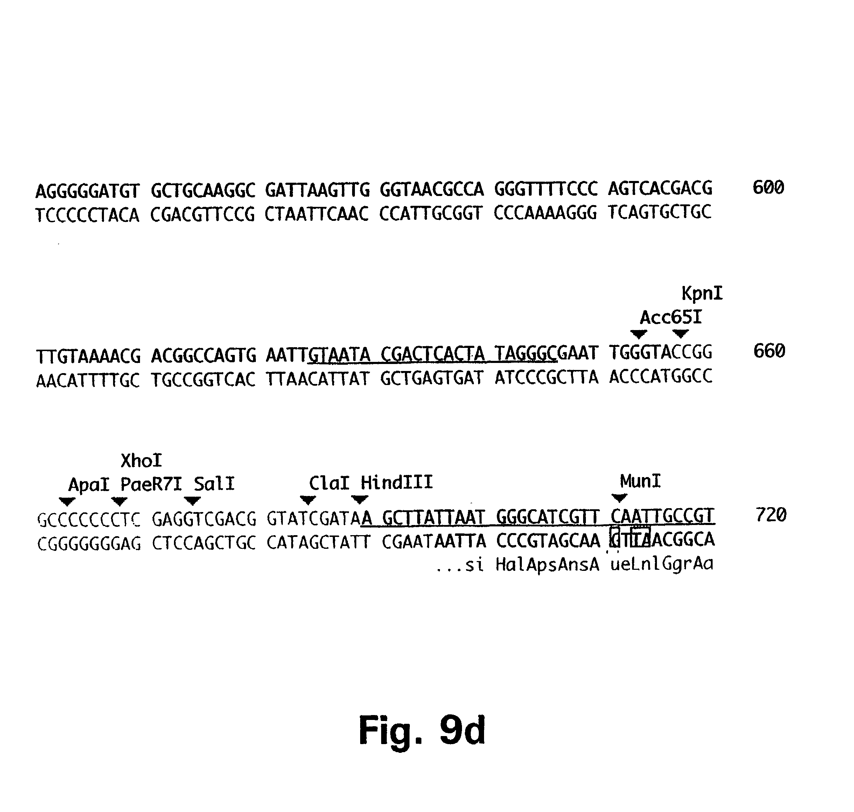

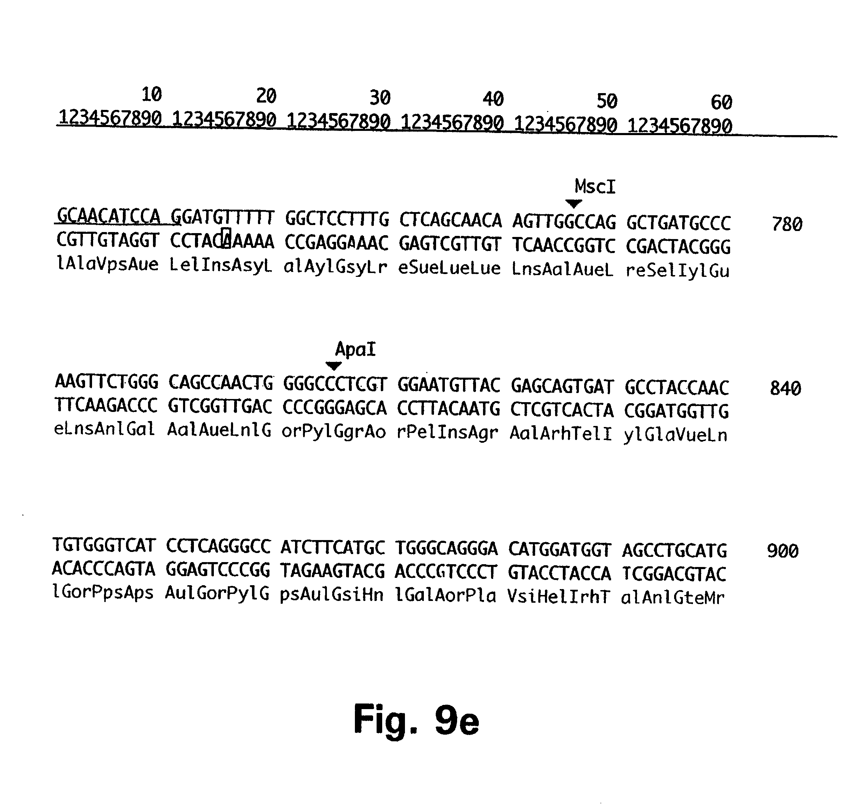

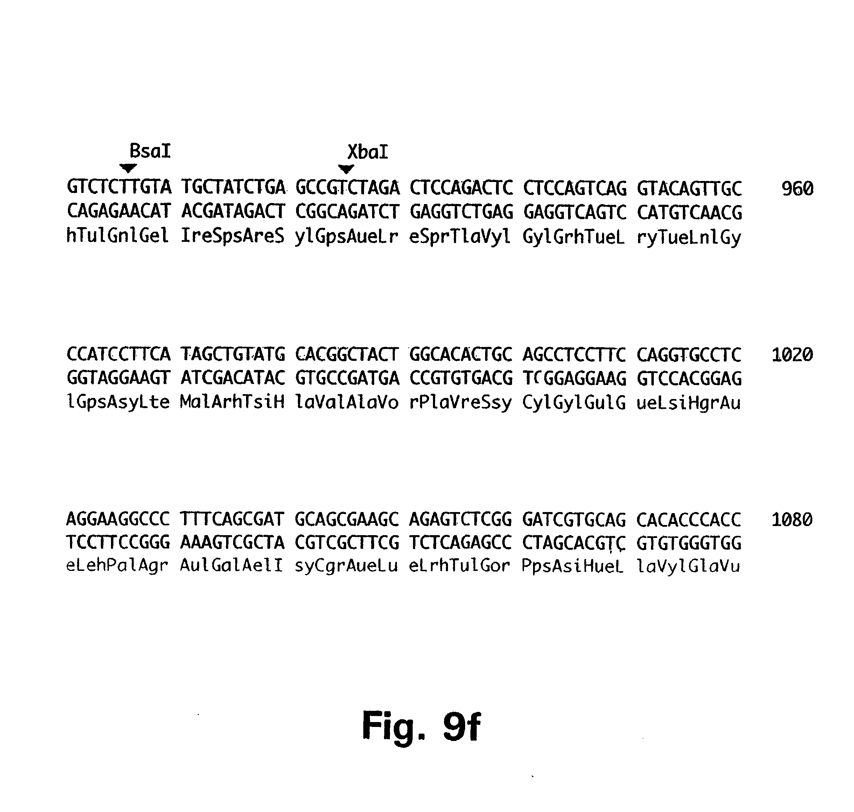

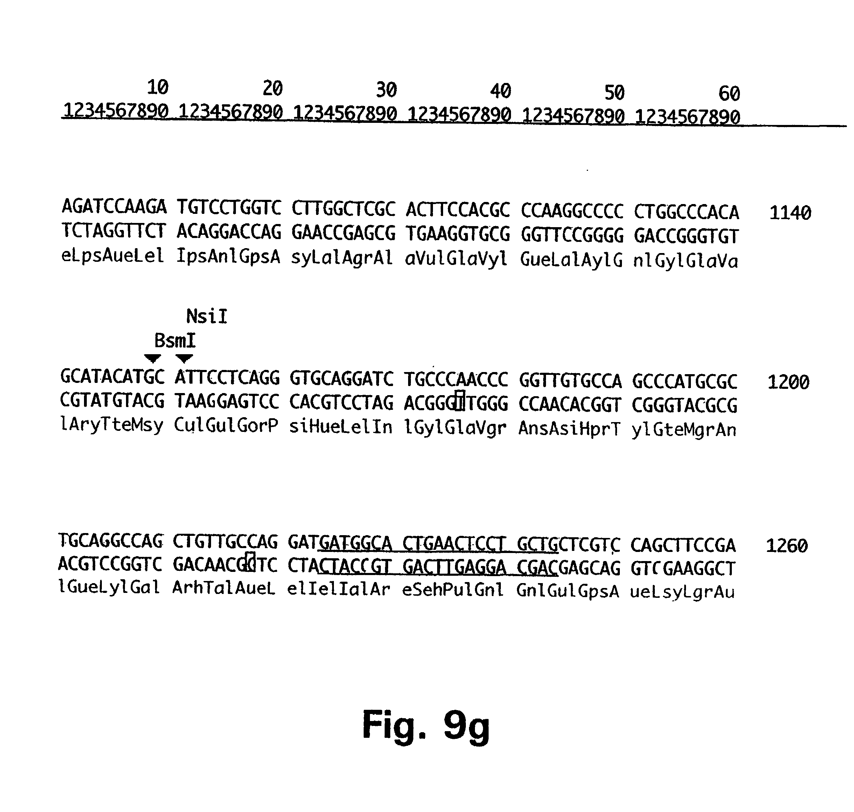

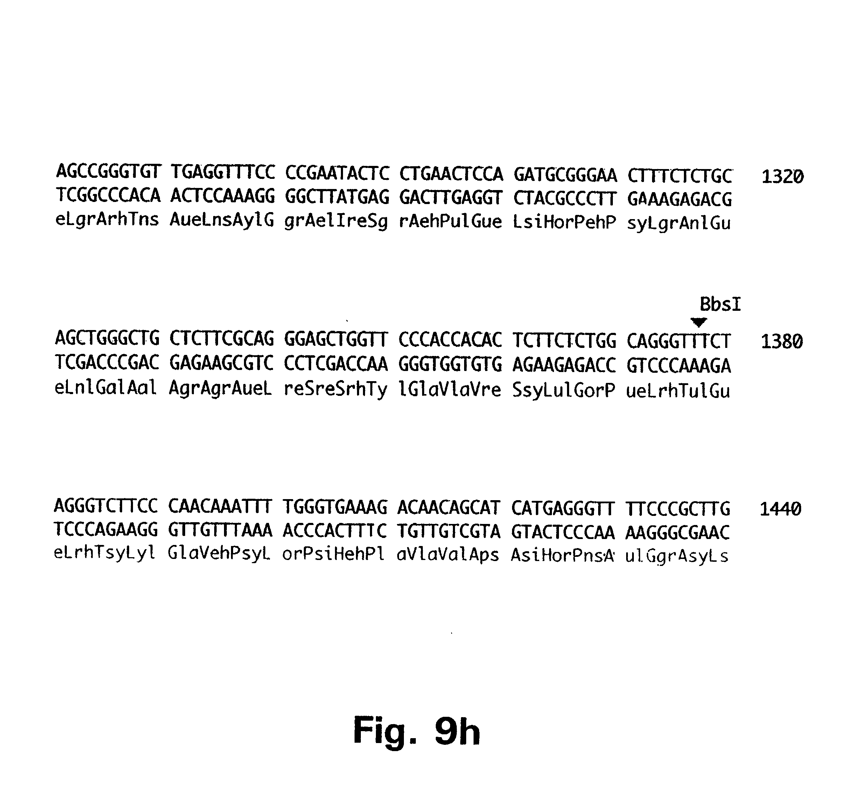

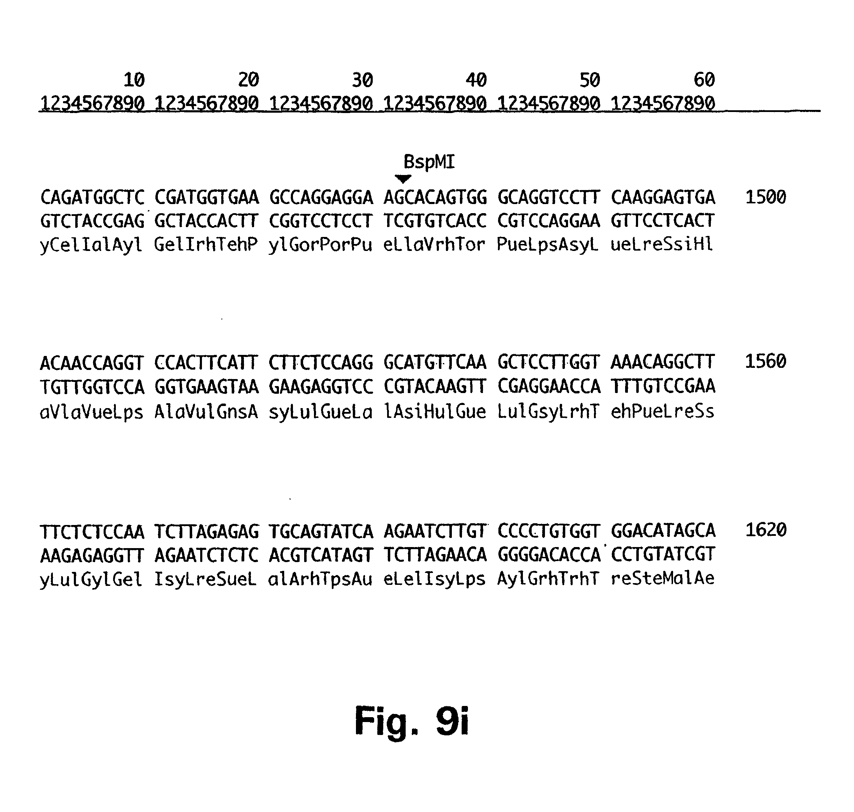

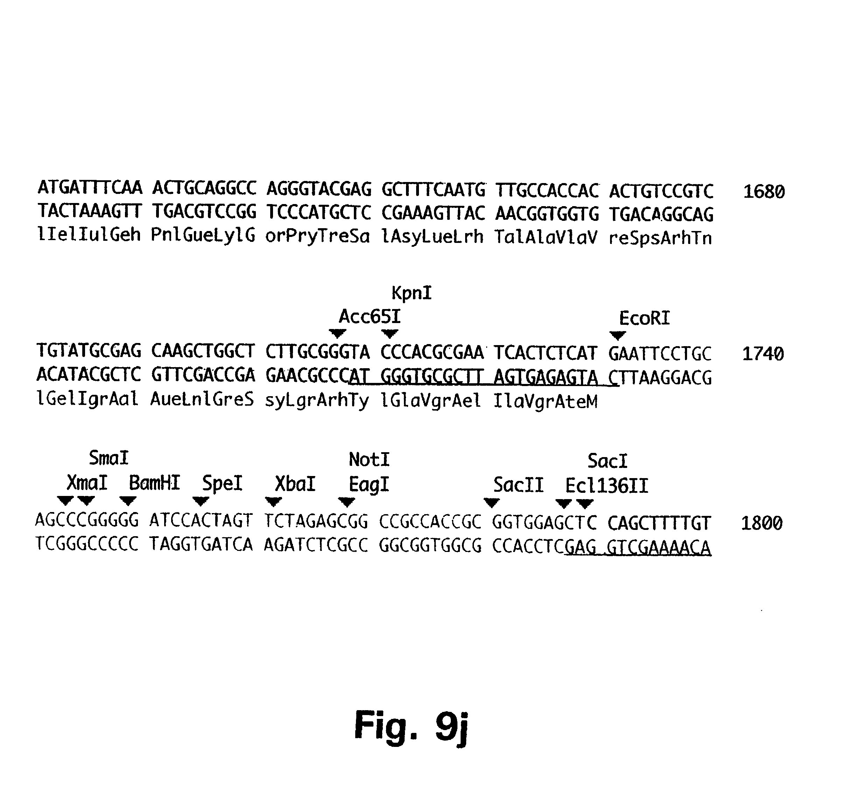

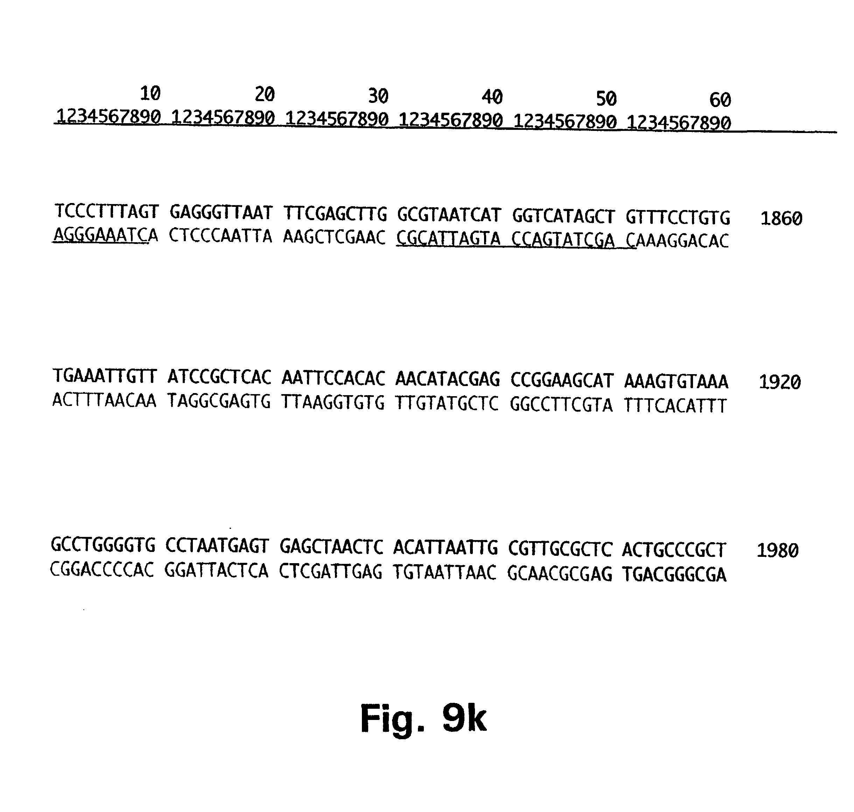

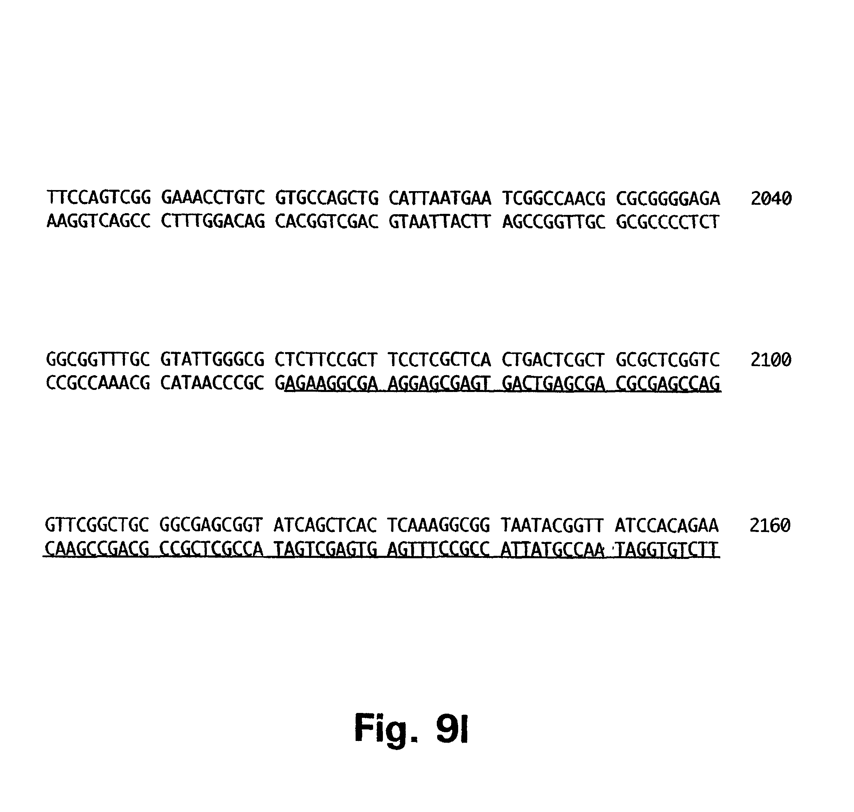

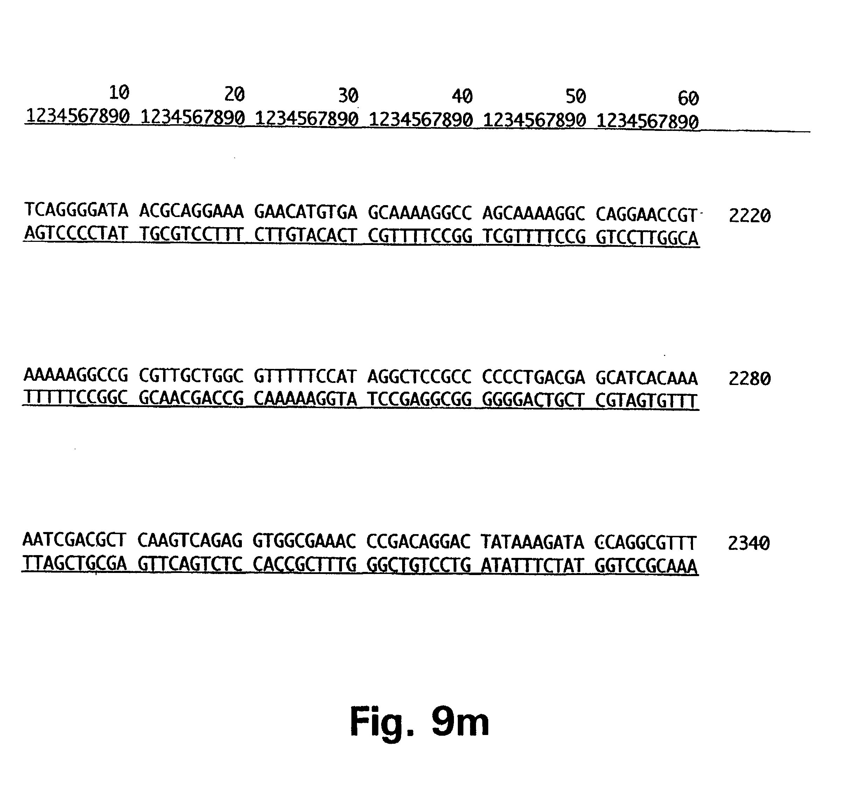

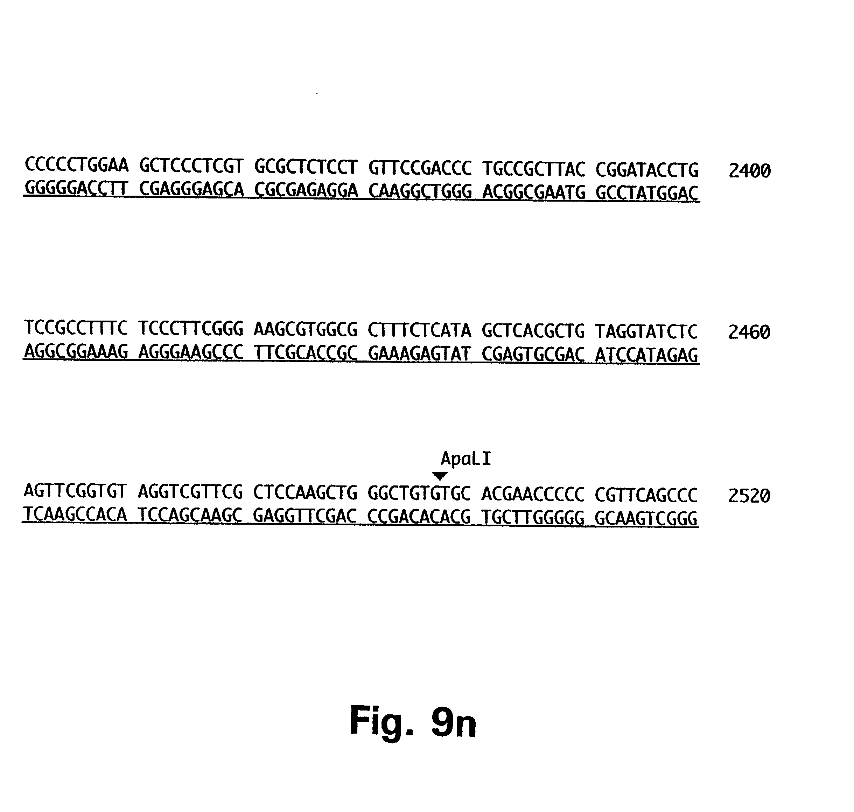

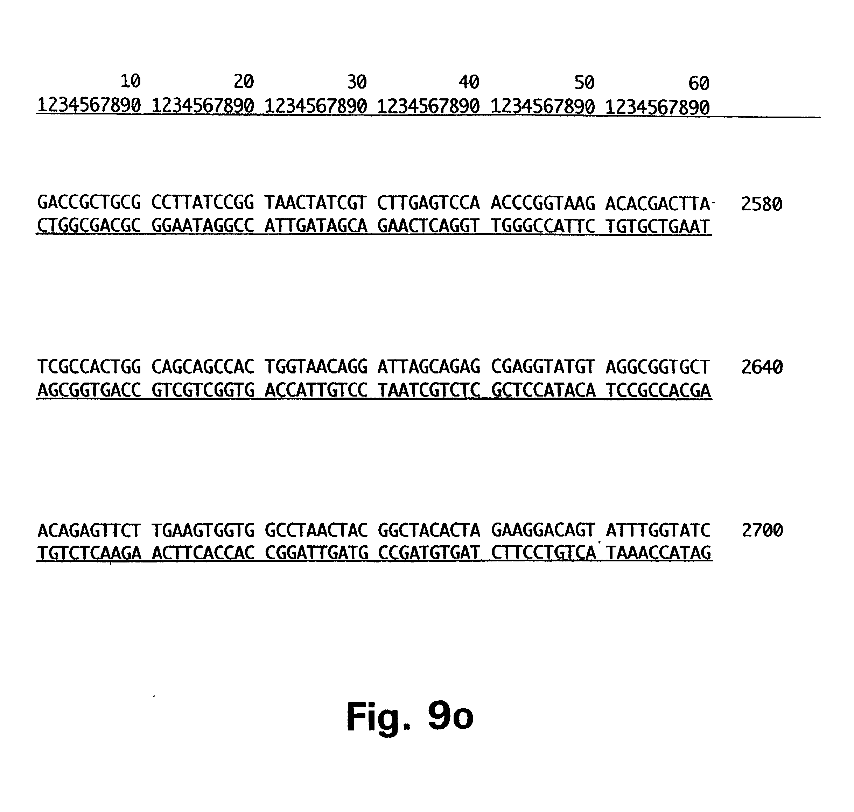

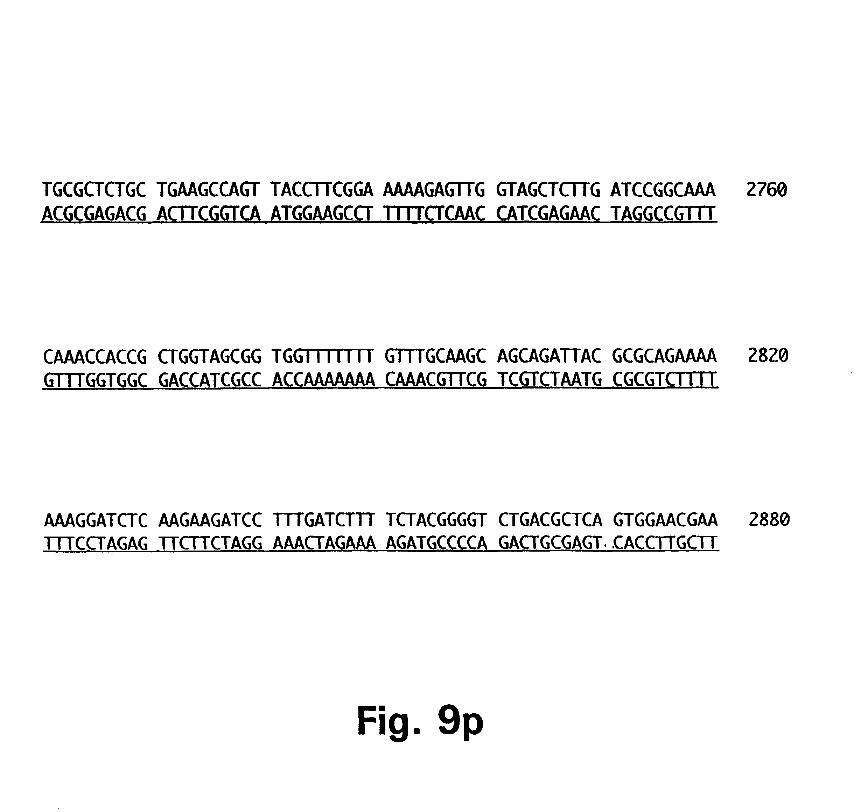

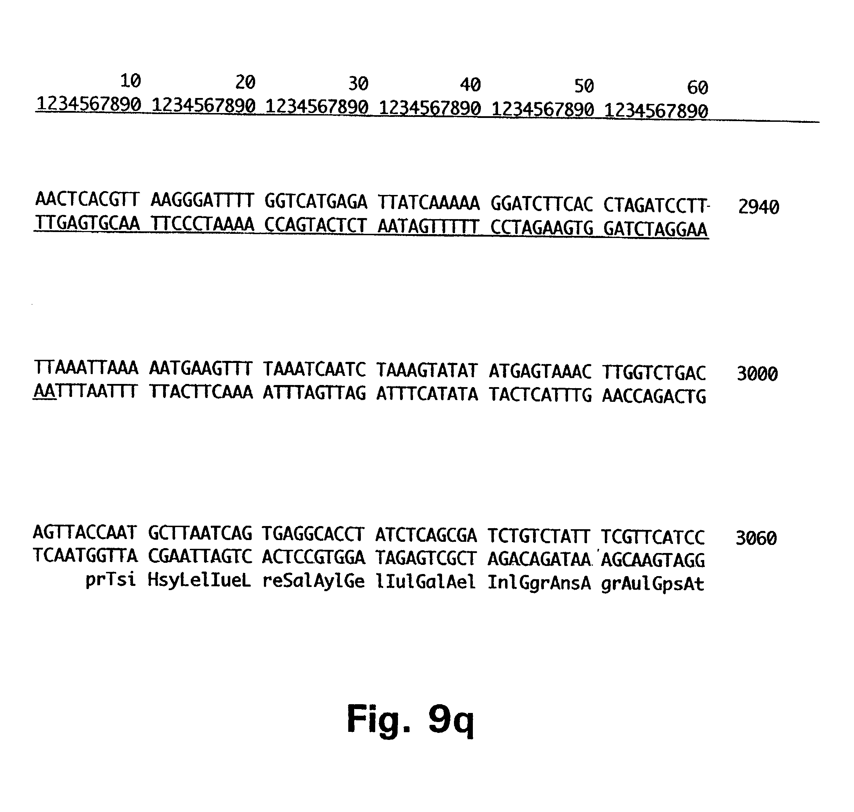

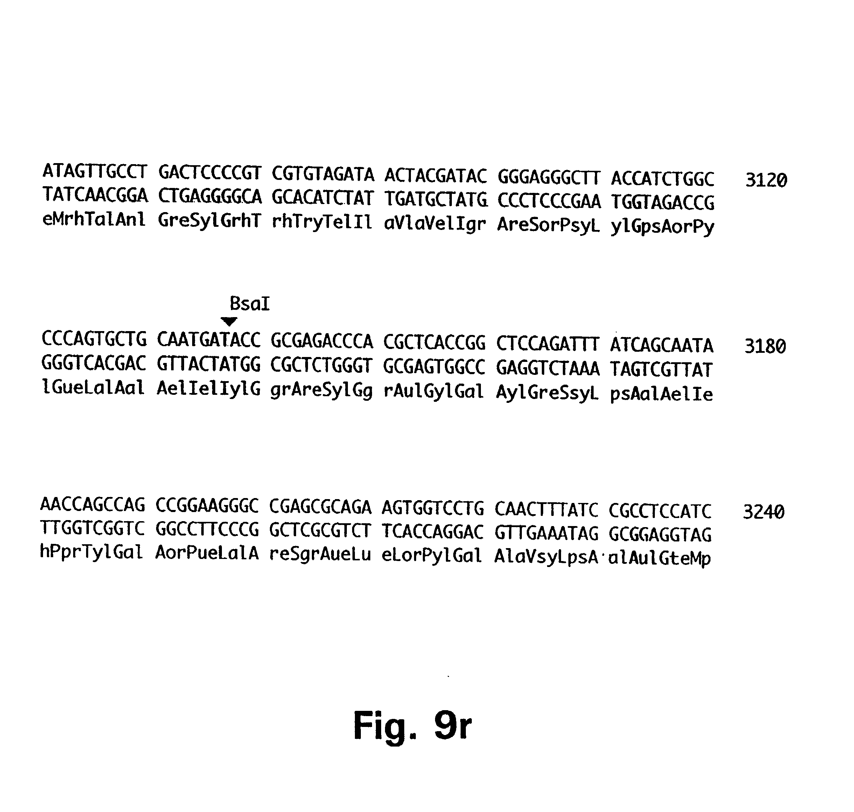

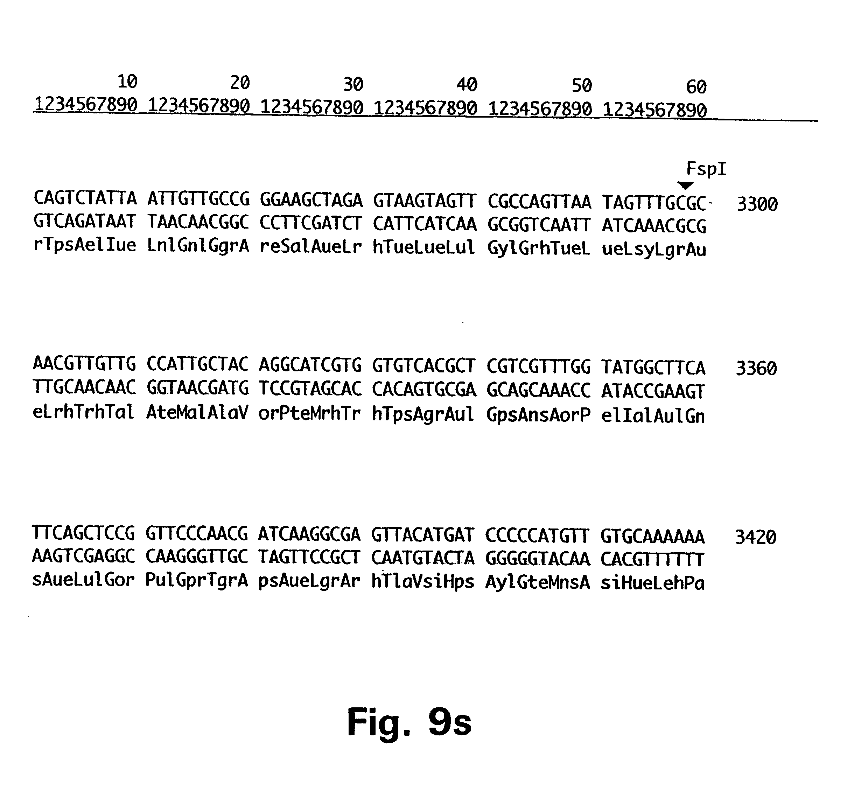

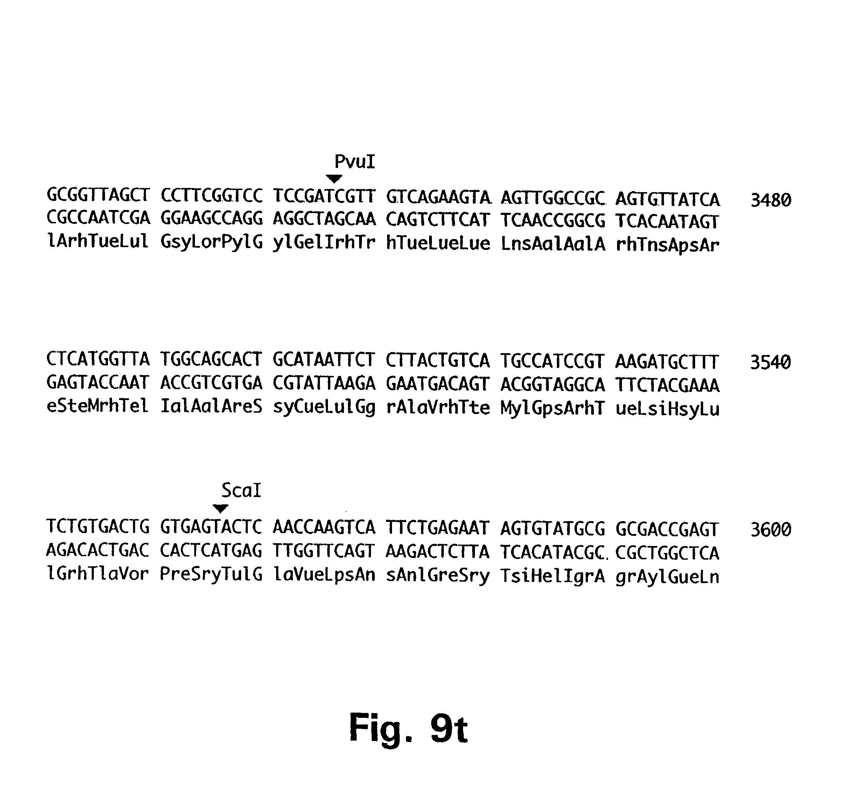

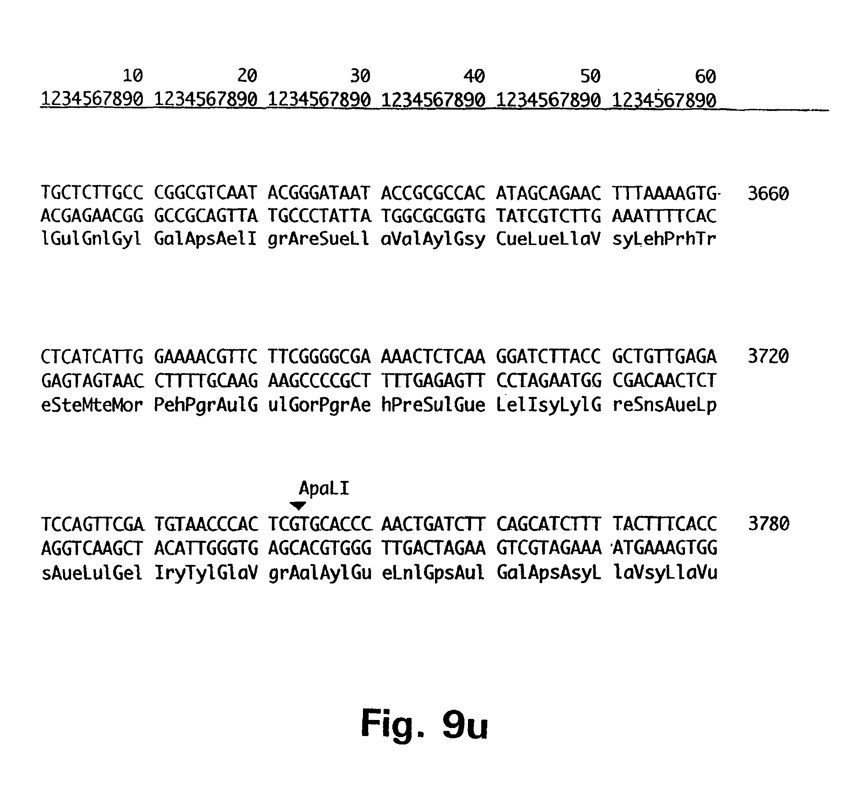

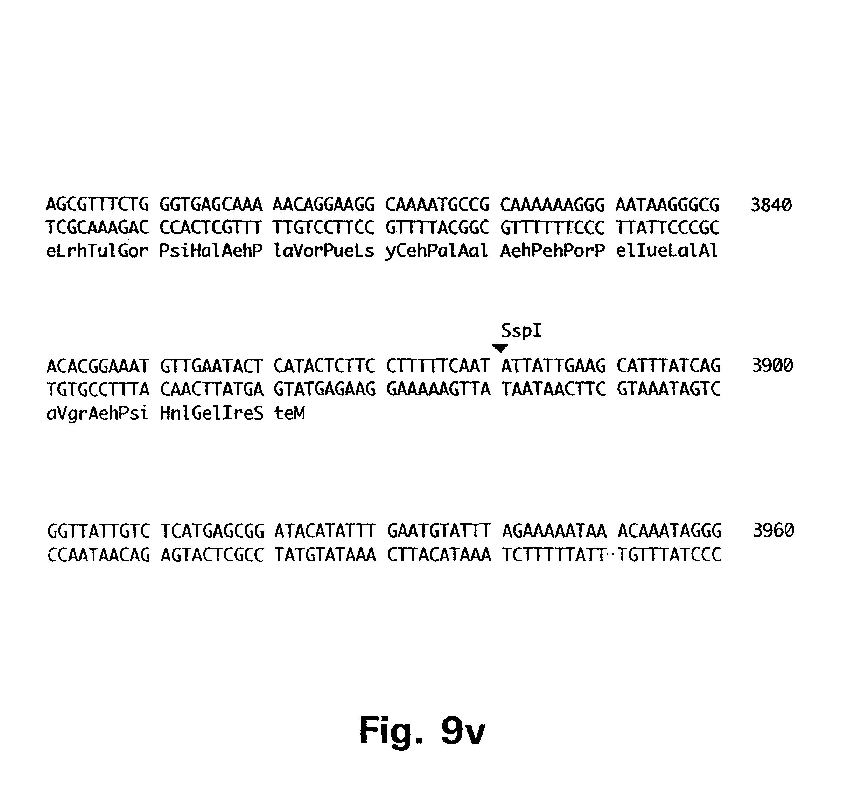

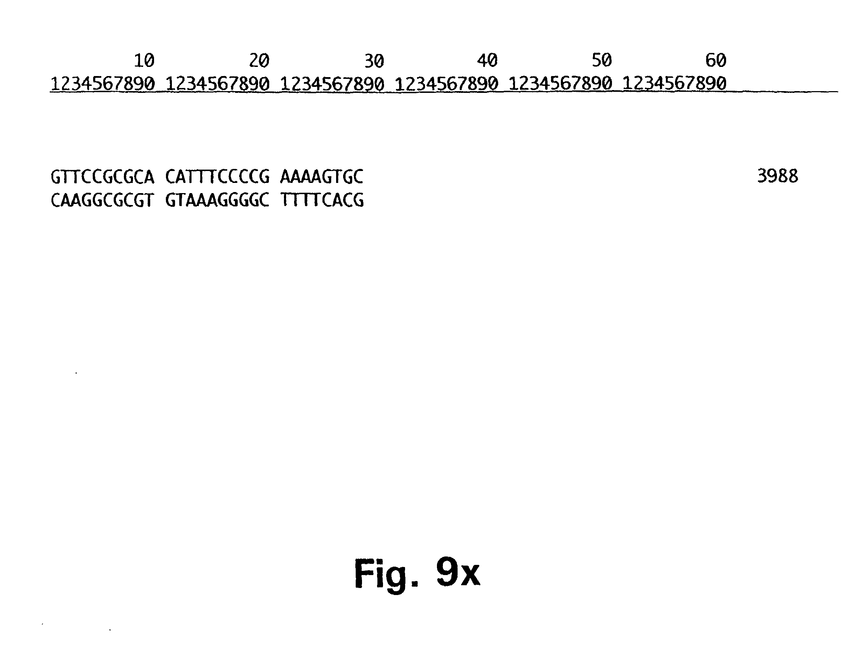

The inserts of all 8 clones were confirmed to be PBGD by complete double strand sequence analysis (see sequences 1 - 8). Each has some change(s) from the published sequences. Some changes are unique and some are shared with other clones (see Table 2 and Table 3). For differences found only in one clone, it is difficult to distinguish between PCR or cloning artifacts and actual allelic variations without additional sampling. However, when the same base difference is found in more than one sequence it is unlikely to be from cloning errors. From the alignment of all 11 PBGD sequences a set of common bases emerged, the consensus or wild type allele sequence. Five of the eight clones (1.1, 1.3, 2.1, 3.3, and 5.3.) have the wild type amino acid sequence. Within this set with wild type amino acid sequence, there is only one difference at the nucleic acid level. At position 555, 4 of the 5 sequences have a dGTP while 1 along with the published erythropoietic and genomic PBGD have a dTTP. These appear to be two common alleles, which result in no amino acid difference. There are 2 base changes between clone number 1.1 and the published erythropoietic PBGD. An adenine to guanine change at base 513 (Leu 171) is a silent mutation, which is also present in 9 out of the 11 sequences, compared. The second difference is a cytosine to adenine substitution at base 995 (Thr 332.) This is not a silent change, with a threonine to asparagine non-conservative mutation. It appears however that the difference is an error in the published erythropoietic PBGD sequence since all 10 other sequences have an adenine at this position. In addition to these natural variations, there are three additional silent mutations introduced during the cloning at positions 1017, 1018 and 1020 to create a Mun-I site for future manipulations. The PBGD gene was ligated into pBluescript SK plasmid generating the pSK-PBGD 3988 bp plasmid, which was sequenced (see Fig. 1, Fig. 9a-9x and sequence 9).

### Conclusion:

For any recombinant therapeutic protein it is important that the wild type allele be used to reduce the potential for immunogenicity. We feel confident through our survey of the literature and analysis of PBGD sequence from different individuals that clone number 1.1 represents the most prevalent "wild type" allele in the population with respect to amino acid sequence. Clone number 1.1 contains the consensus wild type amino acid sequence and differs from the published erythropoietic PBGD sequence by only one amino acid. Because this difference is found in all the other PBGD clones besides the erythropoietic PBGD sequence, it, rather than the published erythropoietic sequence, is deamed to be the prevalent wild type sequence. For this reason PBGD encoded by clone number 1.1 was chosen for production of recombinant human porphobilinogen deaminase (rh-PBGD.)

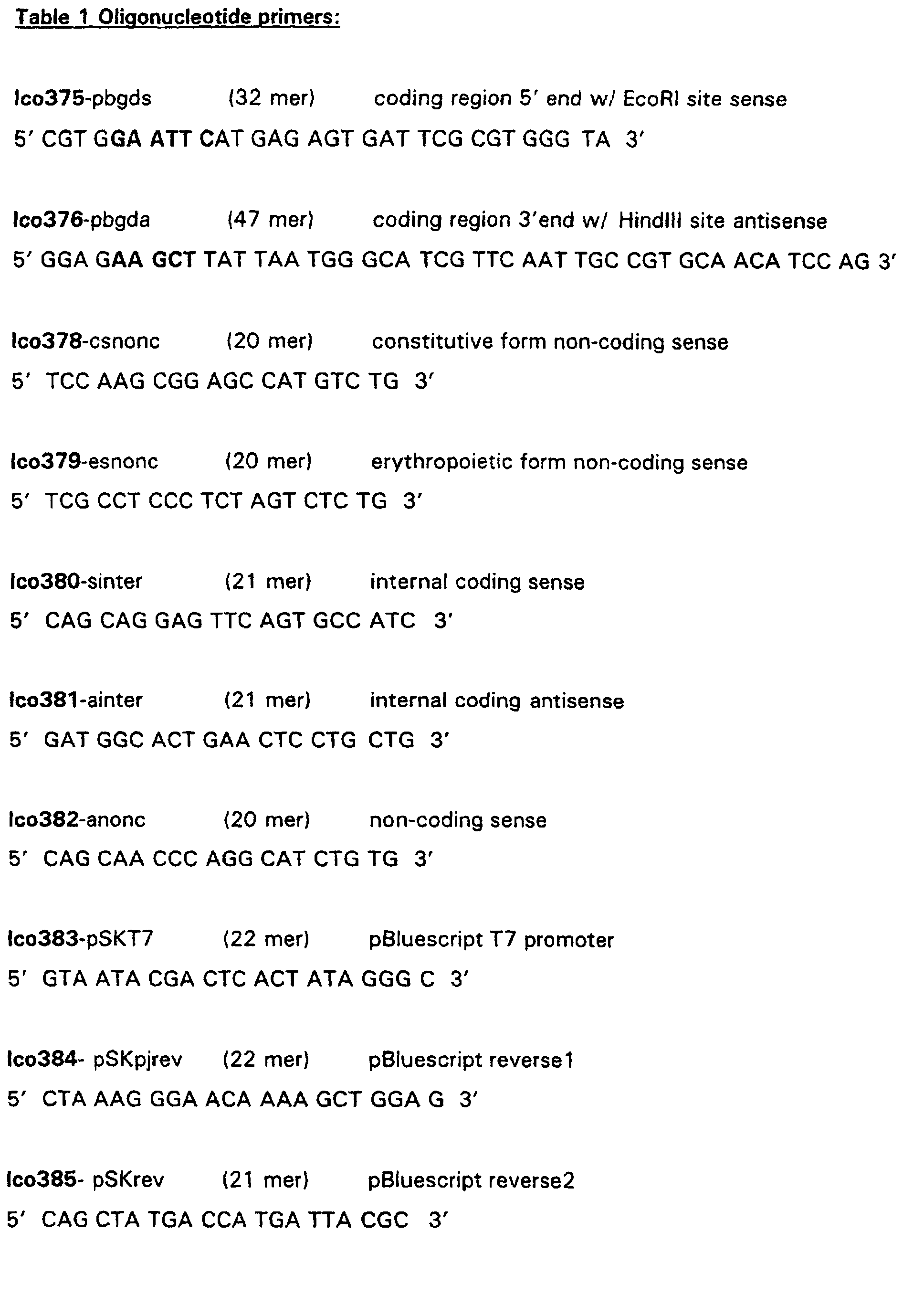

### Equipment list:

| **Item** | **Manufacturer** | **Serial Number** |
|---|---|---|
| Pipetman P-1000 | Gilson | N55287E |
| Pipetman P-200 | Gilson | N52324E |
| Pipetman P-20 | Gilson | N53465M |
| Pipetman P-10 | Gilson | P626586 |
| 5415C centrifuge | Eppendorf | 5415B68381 |
| GS-6KR centrifuge | Beckman | NGD97J18 |
| Avanti J-25 I centrifuge | Beckman | JJY97J14 |
| DU 640B Spectrophotometer | Beckman | 4323015 |
| Genie II vortex | VWR | 2-241186 |
| GeneAmp PCR system 2400 | Perkin Elmer (PE) / Applied Biosystems (ABI) | 803N6021903 |
| GeneAmp PCR system 2400 | PE / ABI | 803S7100104 |
| GeneAmp PCR system 9700 | PE / ABI | 805S7121566 |
| 1545 incubator | VWR | 0902597 |
| heat block 1 | VWR | 0795 |
| heat block 1 | VWR | 0511 |
| Gene Pulser II Apparatus | BioRad | 340BR2745 |
| Pulse Controller Plus | BioRad | 339BR1377 |
| Power Pac 1000 | BioRad | 286BR00770 |
| Sub Cell | BioRad | 16S/8860 |
| Wide-Mini Sub Cell | BioRad | 02S/7951 |
| Foto/Prep transilluminator | Fotodyne | PTG1-0997-2831 |
| Elutrap Electro-separator | Schleicher + Schuell | Order No. 57880 |
| Innova 4000 incubator | New Brunswick Scientific | 890165366 |
| Power Mac G3 computer | Macintosh | XA8061A3BBW |
| Trinitron Multiscan 200GS monitor | Sony | 8057052 |
| DNA analysis Software: Geneworks | Intelligenetics | Version 2.5.1 |

### Supplies List:

| **Item** | **Supplier** | **Cat No.** | **Lot No.** |
|---|---|---|---|
| Human Spleen Poly A+ RNA | Clontech | 6542-1 | 7120266 |
| Human Bone Marrow Poly A+ RNA | Clontech | 6573-1 | 56714 |
| Human Lung Poly A + RNA | Clontech | 6524-1 | 7050104 |
| Human Lymph Node Poly A+ RNA | Clontech | 6594-1 | 6120292 |
| Human Brain Poly A+ RNA | Clontech | 6516-1 | 63101 |
| Human Adipose Total RNA | Clontech | D6005-01 | 7907005 |
| Superscript II reverse transcriptase | Gibco/BRL | 18064-014 | JM6418 |
| 100 mM dNTP set | Pharmacia | 27-2035-01 | 6072035011 |
| pBluescriptII SK- phagemid | Stratagene | 212206 | 0270702 |
| Advantage cDNA polymerase mix | Clontech | 8417-1 | 8060354 |
| GeneAmp dNTP | PE/ABI | N-808-0007 | H0172.4,H0553 |
| Xba-I endonuclease | New England Biolabs (NEB) | 145S | 30 |
| Pvu-II endonuclease | NEB | 151L | 14 |
| EcoR-I endonuclease | NEB | 101L | 25 |
| Hind-III endonuclease | NEB | 104S | 49 |
| Tris six-Pack "C" | Sigma | T-PAC-C | 77H9049 |
| 0.5 M EDTA pH 8.0 | Sigma | E-7889 | 16H8924 |
| Chromaspin TE 400 | Clontech | K1323-1 | 7090795 |
| Chromaspin 400 DepC dH20 | Clontech | K1333-1 | 7040086 |
| Quiaquick gel extraction kit | Qiagen | 28704 | BY97017/0397/11 9 |
| Microcon-30 | Amicon | 42410 | L8JM4330B |
| Microcon-100 | Amicon | 42413 | L8DM3296A |
| Micropure 0.22µm | Amicon | 42544 | CCB017 |
| Seakem GTG agarose | FMC | 50074 | 709397 |
| 100 bp DNA Ladder | NEB | 323-1 | 3 |
| 123 bp DNA Ladder | Gibco/BRL | 15613-029 | JK9706 |
| T4 DNA Ligase | NEB | 202S | 64 |
| Ampicillin | Sigma | A-9518 | 76H0434 |
| LB media | Gibco/BRL | 12795-084 | 12E1072B |
| Bacto Agar | Difco | 0140-07-4 | 106728JA |
| DH10B electromax | Gibco/BRL | 18290-015 | KHN430 |
| SOC media | Gibco/BRL | 15544-042 | 1010351 |
| Taq polymerase | Fisher | FB-6000-15 | H0436 |
| TaqStart antibody | Clontech | 5400-1 | 6070479 |
| Qiafilter Midi DNA isolation kit | Qiagen | 12243 | PO No. 514 |
| Isopropanol | Sigma | I-9516 | 47H3724 |
| Big Dye terminator cycle sequencing kit | PE/ABI | 4303152 | 9803008 |

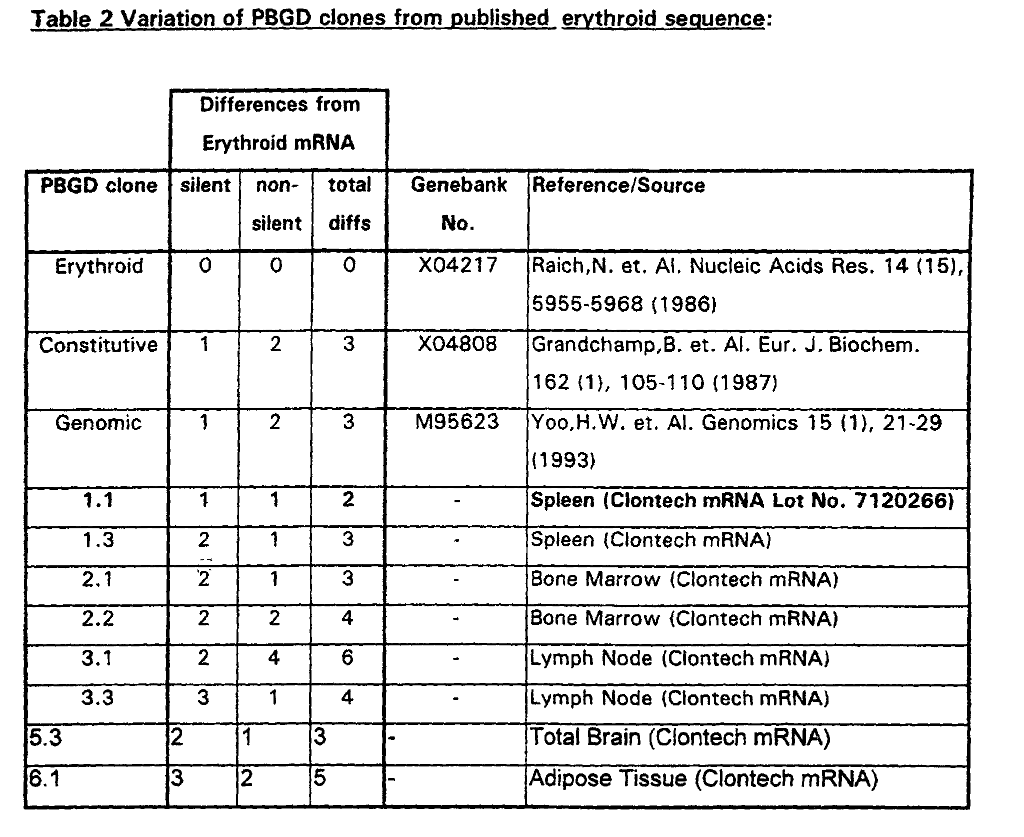

### Table 2:

Summary of the number of differences in amino acid sequence of our sequenced PBGD clones and clones from Genebank entrys for the constitutive and genomic PBGD with published Erythropoietic PBGD sequence. Shown in different columns are the total number of silent mutations with a DNA base change not causing a corresponding amino acid change, the number of non-silent mutations with a DNA change causing an amino acid difference and the sum of the two types of mutations. Not included in this figure are the three silent mutations introduced into the clones to create an internal Mun-I endonuclease cleavage site. Note that clone number 1.1 which will be used for production of recombinant human porphobilinogen deaminase (rh-PBGD) has only one of each type of difference with the least number of total differences.

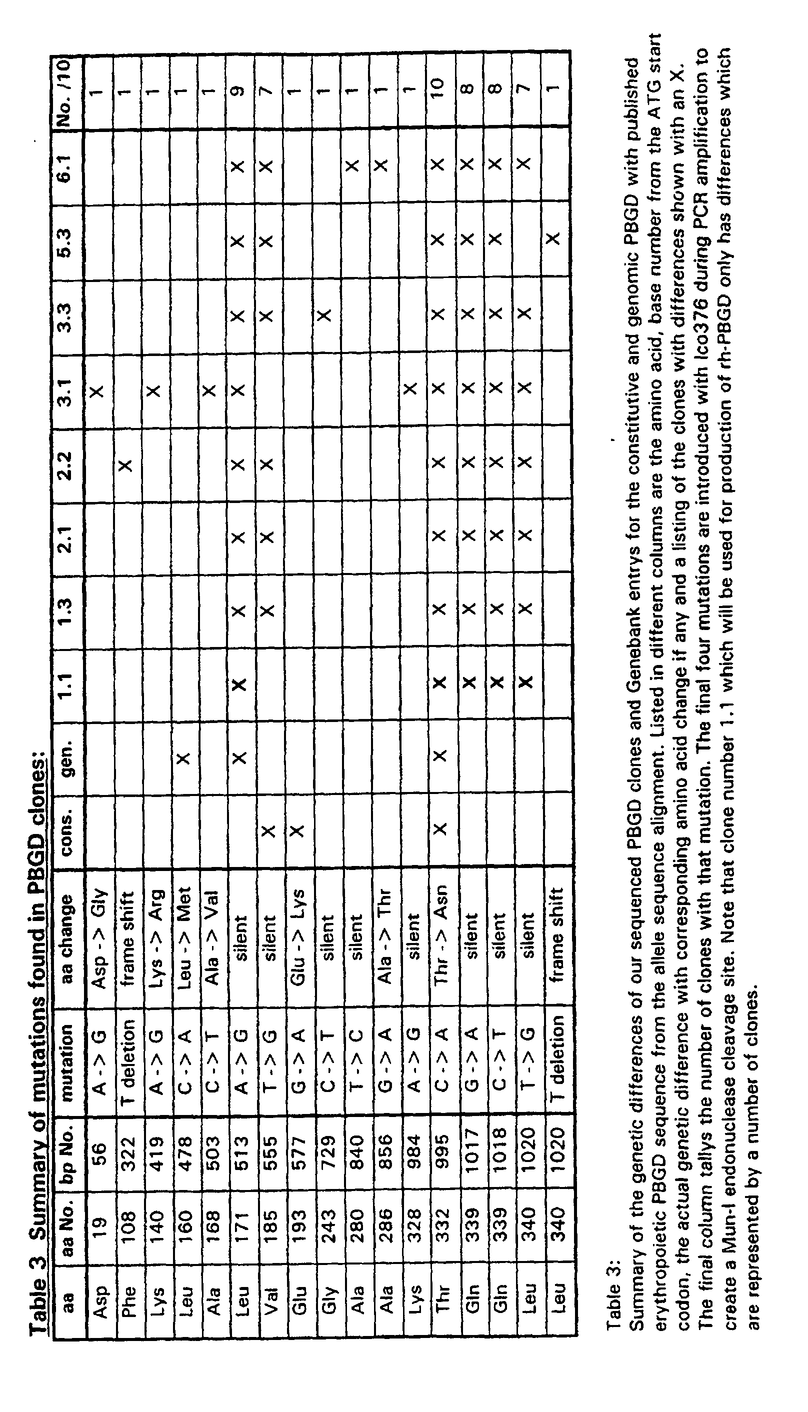

### Expression plasmids

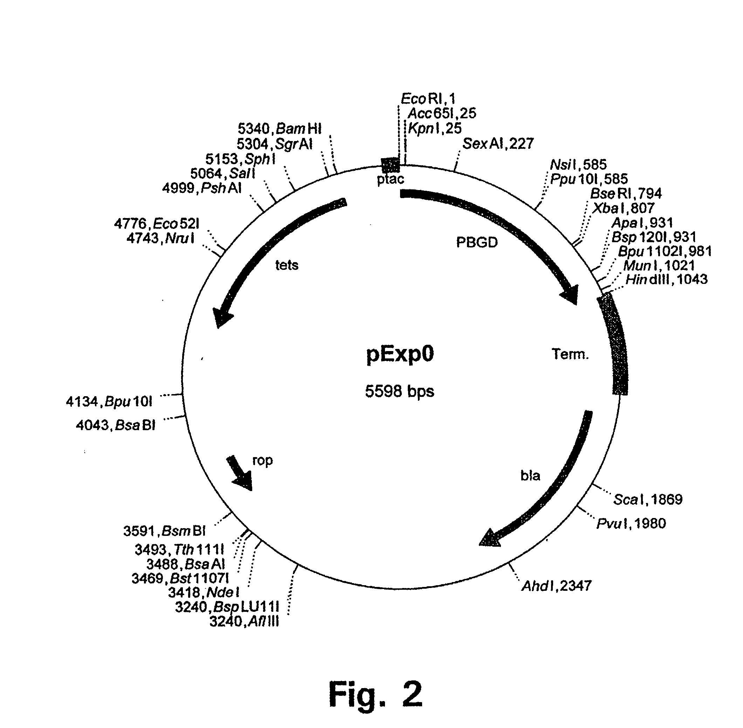

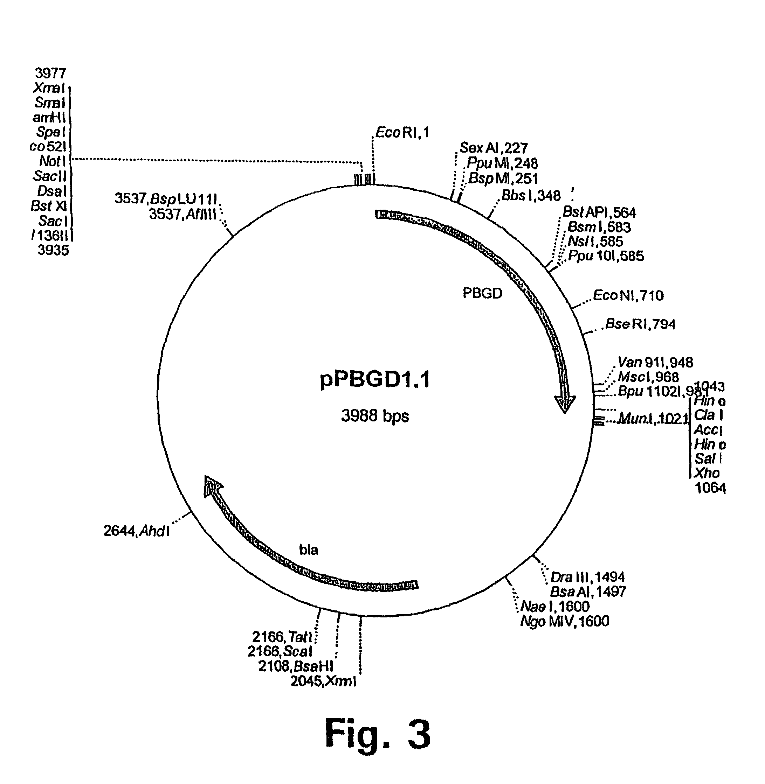

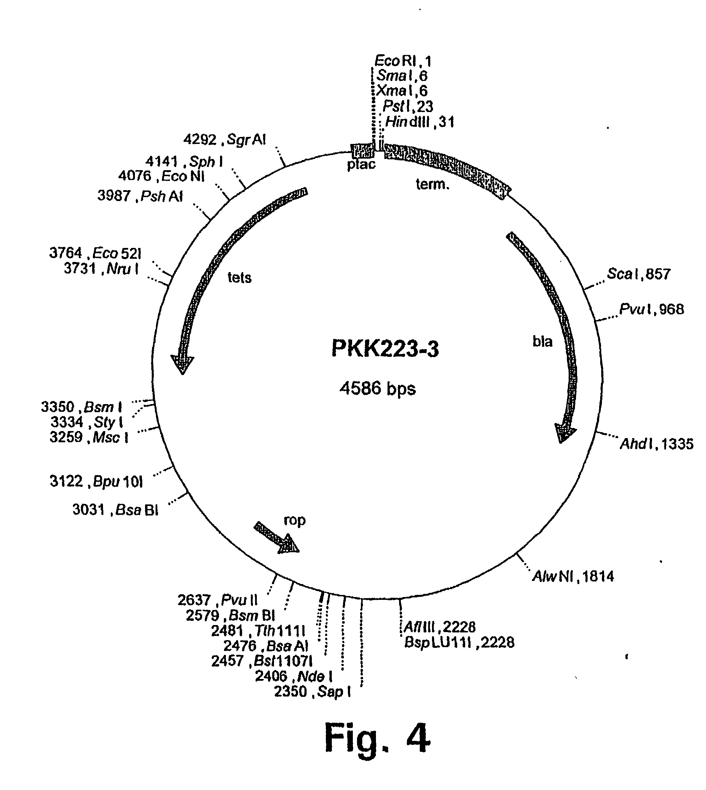

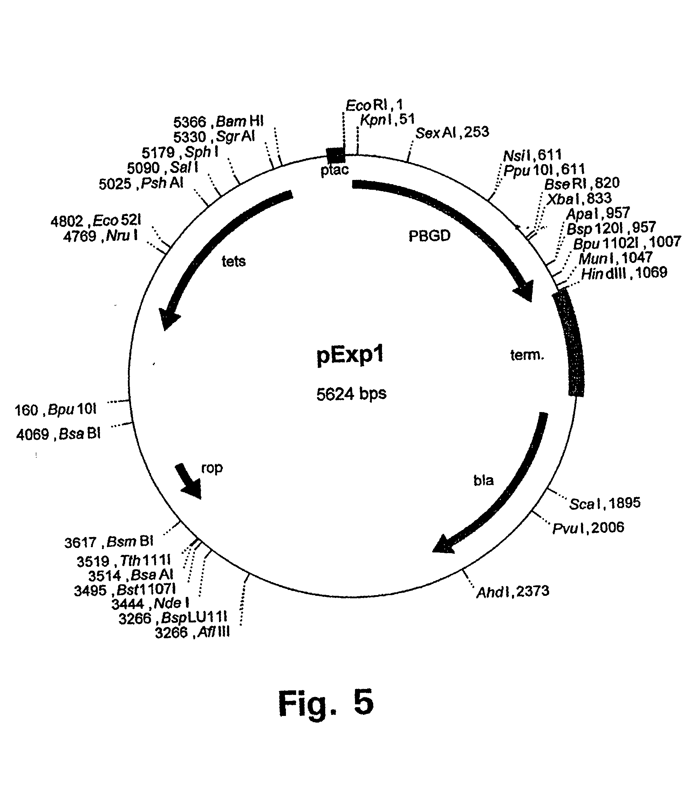

The expression plasmid pExp0 (see Fig. 2) was constructed by excising the PBGD coding sequence (nt1-1043) from pPBGD1.1 (see Fig. 3) with EcoR I and Hind III and inserting it into the vector pKK223-3 (see Fig. 4) (Pharmacia Cat # 27-4935, Lot # 8054935011) cut with the same enzymes, thus operatively linking it to the inducible tac promoter. Another plasmid, pExp1 (see Fig. 5) was also constructed with modifications to the 5' untranslated region and the initial part of the coding sequence both aimed at improving translation efficiencies (translation enhanced). The changes are indicated below and include, insertion of a second ribosome binding site, an AT rich sequence preceding the ATG and three silent base substitutions shown in as underlined.

AATTCTAACA TAAGTTAAGG AGGAAAAAAA A ATG AGA GTT ATT CGT GTC GGT AC
Met Arg Val Ile Arg Val Gly

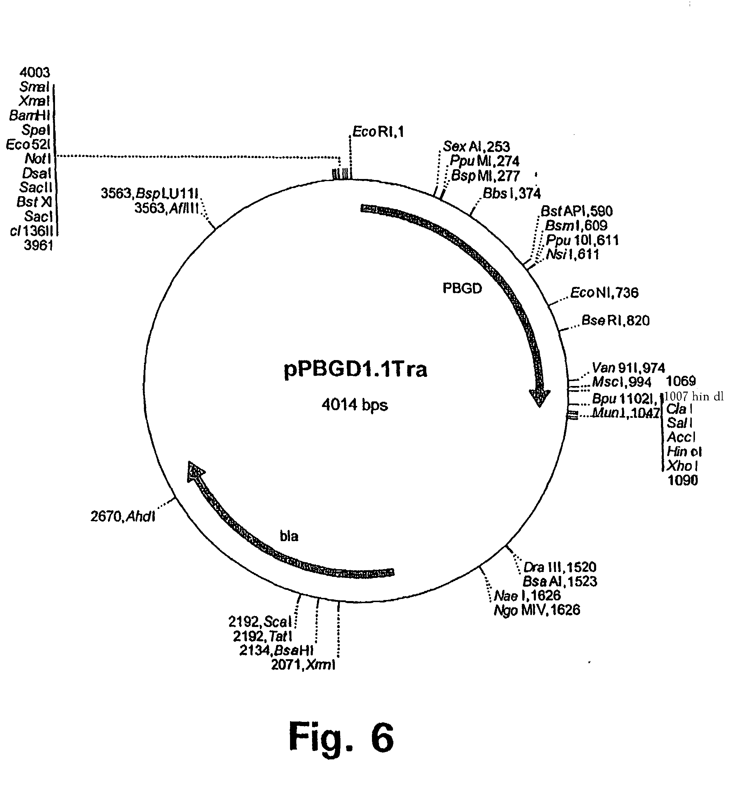

Plasmid pExp1 (Fig. 5) was made in a two step process. Oligonucieotides ICO386 (5' AAT TCT AAC ATA AGT TAA GGA GGA AAA AAA AAT GAG AGT TAT TCG TGT CGG TAC 3') and ICO387 (5' CGA CAC GAA TAA CTC TCA TTT TTT TTT CCT CCT TAA CTT ATG TTA G 3') were designed to provide upon annealing a 5' *Eco*R I adhesive end and a 3' *Kpn* I sticky end. Oligonucleotides ICO386 and ICO387 were annealed and ligated with the *Kpn* I-*Hind* III PBGD fragment from pPBGD1.1 into *Eco*R I -Hind III linearized pBluescript II SK-(Stratagene Cat # 212206) to yield plasmid pPBGD1.1Tra (Fig. 6). In the second step the *Eco*R I -Hind III fragment from pPBGD1.1Tra was ligated into pKK223-3 cut with the same enzymes resulting in plasmid pExp1 (Fig. 5).

### Host strains and growth conditions

For the studies done so far two E. coli K12 strains were used. Both these strains carry the lacl^{q} repressor and are, DH12S (Life Technologies cat # 1832-017) and JM105 (Parmacia Cat # 27-1550-01). The media used was mainly LB Broth (Difco Laboratories Cat # 0446-07-5) containing 100 µg/ml ampicillin. The cultures were grown at 37° C and induced with 4mM IPTG for 3 hours.

The choice of the final strain will partly depend on the induction strategy used (see below). The use of lacits will force the use of strains that do not carry the lacl^{q} repressor. Further, strains deficient in proteases such as La, Clp and the rpoH locus may prove to be beneficial if thermal induction is to be used.

The hemC gene is deleted in the final production strain. A plasmid, which will be used as a tool for this purpose has been constructed.

### Restoration of tetracycline resistance gene

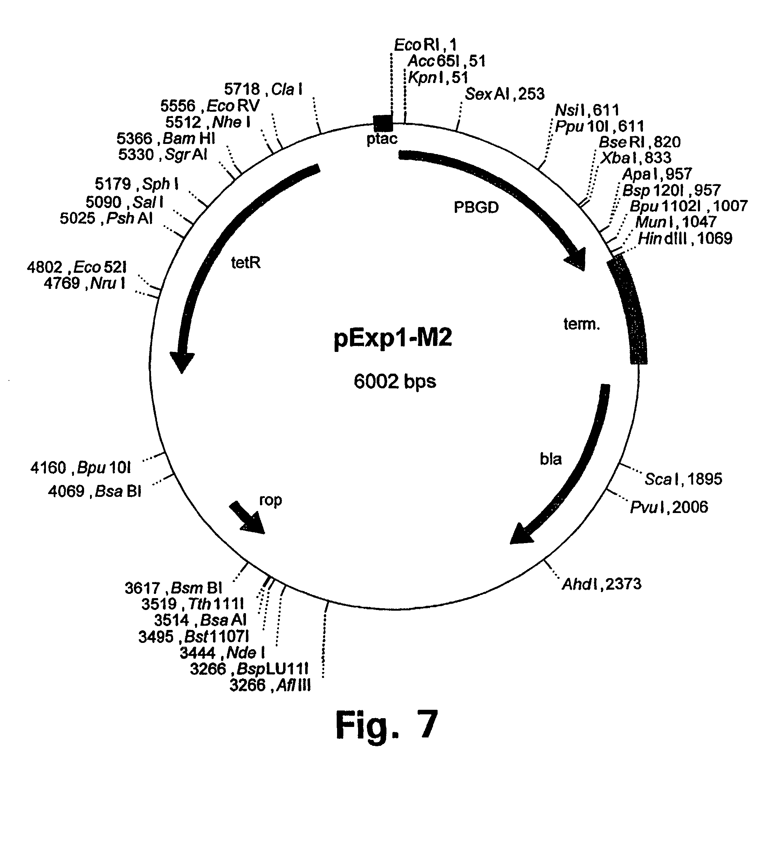

Plasmid pExp1-M2 (Fig. 7) has been constructed from pExp1 with a functional tetracycline resistance gene. This was designed with a view to be able to introduce other regulatory elements or genes as required by the project as also being able to retain the ability to replace the "translation enhanced" PBGD sequence by the native sequence or a polylinker for the blank plasmid.

The following strategy was used. Plasmid pExp1 was cut with *Sal* I and *Bam*H I and the 5348 base-pair fragment containing part of the tetracycline coding sequence and the bulk of the plasmid was isolated. Into this was ligated the Sal I-*Hin*d III fragment from pBR322 (New England BioLabs Cat # 303-3S, Lot # 50) containing rest of the coding sequence and an adapter formed by annealing oligonucleotides ICO424 (5' GATCACTCAT GTTTGACAGC TTATCATCGA TT 3') and ICO425 (5. AGCTAATCGA TGATAAGCTG TCAAACATGA GT 3'). The adapter contains part of the tetracycline promoter and provides *Hind* III and *Bam*H I overhangs for ligation but destroys the *Hind* III and *Bam*H I restriction sites.

### Induction:

The tac promoter is a strong promoter when induced with IPTG and other groups have shown that it or the other almost identical trc promoter can be induced very well at 42°C when a thermosensitive lac repressor is used. With pExp1-M2-Puc-BB construct a high level of expression which can translate to gram quantities of product in the fermenter. Based on study data and data from the literature the thermal induction is efficient.

*Strategy 1:* The plasmid pExp1 (see Fig. 5) has a basal level of expression which is about one-fifth that of a fully induced culture in a lacl^{q} strain in LB broth. Stable and with acceptable expression levels, it can be used without induction in defined fermentation media. In the plasmid pExp1-M2 (see Fig. 7) the rom gene has been deleted on the plasmid and the pBR223 replicon exchanged by the pUC-based replicon. These plasmids have elevated copy numbers with increasing temperature (greater than 30°C) and serve as a pseudo-induction system.

*Strategy 2*: In plasmid pExp1-M2 a thermo-labile lacI repressor has been introduced either at the Sca 1 site or the *Bsa*A 1 site. The repressor was derived from plasmid pTYB1 (New England BioLabs Cat # 6701, Lot # 3) by PCR, wherein Gly¹⁸⁷ was changed to Ser. If the thermal induction works but a high basal level of expression leads to instability, the native PBGD sequence as in pExp0 (without the enhancements) is substituted. In plasmid pExp0 the uninduced level of expression is below the level of detection in the assay system used. Other variations would be to use lacl^{q}ts on the plasmid or in the genome.

### Detailed Description of the Construction of the production plasmid for rPBGD

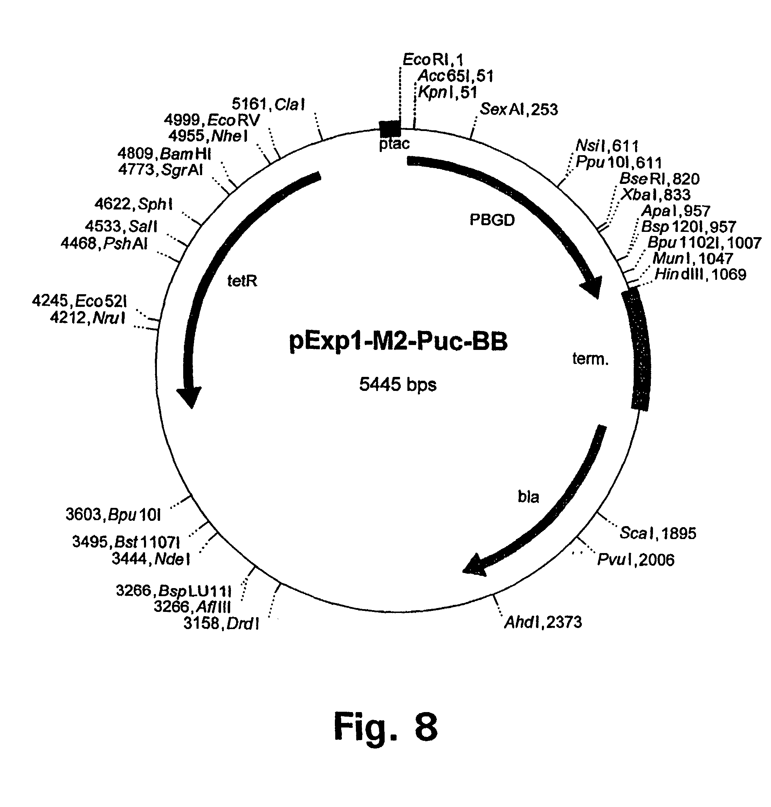

The production plasmid pExp1-M2-Puc-BB (see Fig. 8) for rPBGD was constructed in a multi-step process. The individual steps used and all the intermediate plasmids are outlined below.

The expression plasmid pExp1 was first constructed wherein the PBGD coding sequence is operatively linked to the tac promoter of the vector pKK223-3 (Pharmacia Cat # 27-4935, Lot # 8054935011) by means of a short adapter designed to include a ribosome binding site, an AT-rich region preceding the ATG and the first six codons of the PBGD human erythropoietic enzyme with three silent base changes. The sequence of the adapter is shown below with the base changes introduced in the wobble positions boldfaced. 
AATTCTAACA TAAGTTAAGG AGGAAAAAAA A ATG AGA GTT ATT CGT GTC GGT AC
Met Arg Val Ile Arg Val Gly
 Oligonucleotides ICO386 (5' AAT TCT AAC ATA AGT TAA GGA GGA AAA AAA AAT GAG AGT TAT TCG TGT CGG TAC 3') and ICO387 (5' CGA CAC GAA TAA CTC TCA TTT TTT TTT CCT CCT TAA CTT ATG TTA G 3') were designed to provide upon annealing a 5' *Eco*R I adhesive end and a 3' *Kpn* I sticky end. Oligonucleotides ICO386 and ICO387 were annealed and ligated with the *Kpn* I-*Hind* III PBGD fragment from pPBGD1.1 into *Eco*R I-*Hind* III linearized pBluescript II SK- (Stratagene Cat # 212206) to yield plasmid pPBGD1.1Tra. In the second step the *Eco*R I *-Hind* III fragment from pPBGD1.1Tra was ligated into pKK223-3 cut with the same enzymes resulting in plasmid pExp1.

The teracycline resistance gene was next restored using the following strategy.

Plasmid pExp1 was cut with *Sal* I and *Bam*H I and the 5348 base-pair fragment containing part of the tetracycline coding sequence and the bulk of the plasmid was isolated. Into this was ligated the Sal I-*Hin*d III fragment from pBR322 (New England BioLabs Cat # 303-3S, Lot # 50) containing rest of the coding sequence and an adapter formed by annealing oligonucleotides ICO424 (5' GATCACTCAT GTTTGACAGC TTATCATCGA TT 3') and ICO425 (5. AGCTAATCGA TGATAAGCTG TCAAACATGA GT 3'). The adapter contains part of the tetracycline promoter and provides *Hind* III and *Bam*H I overhangs for ligation but destroys the *Hin*d III and *Bam*H I restriction sites. The resulting plasmid was called pExp1-M2.

Plasmid pExp1-M2 was digested with Pvu I and Afl III and the larger of the two fragments corresponding to a size of 4745 base-pairs was isolated. This was ligated to the 1257 base-pairs long Pvu I-AflIII fragment derived from pUC19 containing the origin of replication and part of the ampicillin resistance gene to obtain plasmid pExp1-M2-Puc. This was passaged through JM110 and cut with BsaA1 and BsaB1 to excise the rom gene contained between the two sites and blunt-ended together to yield the final expression plasmid pExp1-M2-Puc-BB. The pExp1-M2-Puc-BB plasmid has been fully sequenced (see sequence 11).

### Expression of rPBGD in E. coli

The *E. coli* K12 host strain JM105 genotype endA thi rpsL sbcB15 hsdR4 Δ(lac-proAB) [F'traD36 proAB lacl^{q} Δ(lacZ)M15] containing the expression plasmid pExp1-M2-Puc-BB was grown in LB broth containing 100 µg/ml ampicillin at to mid-log phase at 30°C from a 1 to 40 dilution of an overnight inoculum. The culture was then split into three and growth was continued for another 4 hours at 30°C, 37°C and 42°C respectively. Cells were spun down from 1 ml samples and frozen at -20°C. The thawed cell pellets were resuspended in 200-300 µl of B-PER reagent PIERCE Cat. # 78243, incubated at room temperature for 10 minutes, spun at 16,000 for 10 minutes and PBGD activity was determined in the supernatants. Total protein was estimated by the Bradford method using the BioRad reagent Cat # 500-0006 and bovine serum albumin as stsndard. The specific activities in the crude lysates obtained at the three growth temperatures are tabulated below. The results clearly show an increase of PBGD units/mg with increasing temperature in the range from 30°C to 40°C.

| **Temperature** | **PBGD Units/mg** |
|---|---|
| 30°C | 363 |
| 37°C | 573 |
| 42°C | 1080 |

### OTHER PRODUCTION SYSTEMS FOR rPBGD

For yeast production, the PBGD coding sequence can be inserted into a plasmid vector, for example YEP type, containing 2 u circular DNA (Ori) origin for high expression in yeast. YEP plasmids contain TRP 1 and URA 3 as markers for selective maintenance in trp1-, ura 3-yeast strains.

Alternatively, the PBGD gene can be inserted in bovine papilloma virus vectors BPV for high expression in a murine cell line C-127 (Stephens P.E. et. al. Biochem J. 248, 1-11, 1987) or vectors compatible with expression in CHO cells or COS cells.

An expression of PBGD can be made intracellularly.

A secretory signal in Saccharomyces, for example alpha-mating factor presequence, can be added in front of the rPBGD structural gene for efficient secretion in yeast.

Similarly, a sequence encoding a mammalian signal peptide can be added for secretion of rPBGD into the culture medium upon expression in for example CHO cells or COS cells.

A bacterial promoter for example the tryptophane (trp) promoter or the lac promoter or alternatively an alkaline phosphatase promoter, should be inserted before the PBGD coding region for efficient transcription in prokaryotes for example E. coli.

A yeast promoter for example 3-phosphoglycerate kinase (PGK) or chelatin or alpha-mating factor should be inserted before the PBGD coding region for efficient transcription in yeast for example Saccharomyces cerevesiae or Saccharomyces pombe.

A mammalian promoter for example Metallothionin-1 (MT-1) or Aspartate transcarbamylase or Dihydrofolate reductase (DHFR) should be inserted before the PBGD coding region for efficient transcription in mammalian cell lines for example CHO cells or COS cells.

The yeast plasmid (Y-G&F-PBGD) containing a yeast promoter, signal and/or ATG codon (methionine) in front of the PBGD coding region and a yeast vector containing selectable markers such URA 3 or TRP 1 will be transformed into the yeast host cell such as Saccharomyces cerevesiae or Saccharomyces pombe for production of rPBGD.

The mammalian plasmid (M-G&F-PBGD) containing a mammalian promoter for example Metallothionine-1 or Dihydrofolate reductase and a mammalian signal sequence or an ATG codon in front of the PBGD coding region and vector pAT or pSV2 respectively. Plasmid (M-G&F PBGD) will be transfected into a mammalian cell line for example CHO cells, for production of rPBGD.

The E. coli cell containing plasmid (pExp1 or pExp1-M2 Puc-BB), will be fermented to stationary phase between 24-48 hours, in a medium containing casein hydrolysate, or yeast extract, glucose, vitamins and salts. pH oxygen will be monitored by electrodes during fermentation. Temperature will be kept at 37 +/- 2 C during the fermentation.

The yeast cell containing the plasmid (Y-G&F-PBGD), will be fermented to late log phase between 20-40 hours in a medium containing yeast extract, glucose, salts and vitamins. pH and temperature will be monitored by specific electrodes during fermentation. Temperature will be kept at 30+/- 2 C during fermentation.

The mammalian cell line containing the plasmid (M-G&F-PBGD) will be fermented in a medium containing, foetal calf serum (or serum free), vitamins, glucose, antibiotics, growth factors. pH and temperature will be monitored continuously during fermentation by specific electrodes.

### Fermentation and Purification

rPBGD will be recovered from E. coli after fermentation by an extraction procedure involving for example ribipress, sonication, osmotic shock or total solubilization by detergent for example Tween 80, Triton X-100 or Brij. rPBGD will be recovered from fermentation medium after production in yeast or from a total cellular extract using detergents such as Triton X-100, Tween 80 or Brij. rPBGD will be recovered from mammalian culture medium or from a total cellular extract by ion-exchange chromatography or affinity chromatography.

rPBGD will be purified from E. coli extract or from yeast medium or total cellular extract or from mammalian culture medium or total mammalian cellular extract by binding to an ion-exchange column for example DEAE-Sepharose or MonoQ-Sepharose and eluted with for example NaCI and Sodium phosphate buffer pH 7-8 or the corresponding potassium salts.

Alternatively, rPBGD will be recovered from extracts by binding to an affinity chromatography column for example an anti-PBGD affinity column. rPBGD will be eluted by lowering the pH to 4-2, or a thiol specific affinity column. rPBGD has been "tagged" with thiols residues when a thiol affinity column step is used. Thiols will be removed by a specific enzymatic cleavage step to generate authentic rPBGD.

The ion-exchange or affinity purified rPBGD will be further purified by hydrophobic interaction chromatography on for example, TSK Phenyl 5 PW column or Octyl-Sepharose or Phenyl-Sepharose columns.

Binding of rPBGD will be done at high ionic strength for example in 10-50 mM Tris-HCl pH 7-8, 1M NaCI or 10-15 mM Sodium phosphate pH 7-8, 0.5 M MgSO₄ and eluted by lowering the ionic strength for example with 10-50 mM Tris-HCI pH 7-8 or 10-50 mM Sodium phosphate pH 7-8.

Three hydrophobic interaction steps will be applied consecutively.

rPBGD will be further purified with preparative RP-HPLC for example C12 or C18 matrixes. The rPBGD will be eluted from the column by a gradient of 10-50 mM Sodium phosphate and 1-10% acetonitrile buffer.

Formulation of rPBGD will be done by passing the enzyme over a G-100 Sephadex column and eluting it in an isotonic solution for example 0.9%NaCl and 10-50mM Sodium phosphate pH7.0 +/- 0.5 or Sodium phosphate, glycin, mannitol or the corresponding potassium salts.

The formulation solution of rPBGD will be sterile filtered and filled aseptically in glass vials and lyophilised.

Alternatively, the sterile filtered rPBGD solution will be formulated in for example, lipid vesicles constituting phosphatidylcholine or phosphatidylethanolamine or combinations of these or incorporated into erythrocyte ghosts.

Reconstitution of lyophilised rPBGD will be done in water for injection.

Alternatively, rPBGD will be formulated in a sustained release formulation involving biodegradable microspheres, for example in polylactic acid, polyglycolic acid or mixtures of these.

Alternatively, rPBGD will be lyophilized in a two-compartment cartridge, where rPBGD will be in the front compartment and water for reconstitution in the rear compartment. This two compartment cartridge will be combined with an injection device to administer either rPBGD by a needle or needle less (by high pressure) device.

Alternatively, rPBGD will be formulated in a physiological buffer containing an enhancer for nasal administration.

Alternatively, rPBGD will be formulated in an oral formulation containing for example, lipid vesicles (phospatidylcholine, phosphatidylethanolamine, sphingomyeline) or dextrane microspheres.

Although recombinant production of PBGD is preferred for the treatment of AIP, it can alternatively be produced from human red blood cells.

The production and manufacturing of recombinant PBGD will be done by the following steps.

### Recombinant PBGD production process; an outline

### A: Fermentation

1. Master cell bank
2. Working cell bank
3. Production of seed culture
4. Fermentation in large fermenter (250 L >)

### B. Purification

1. Cell concentration by filtration/centrifugation
2. Cell disruption
3. Ultrafiltration
4. Chromatography ion exchange, DEAE-Sepharose, MonoQ-Sepharose
5. Hydrophobic interaction chromatography (Octyl/phenyl-Sepharose, TSK Phenyl, 5PW, Phenyl -Sepharose
6. Chromatography ion exchange (MonoQ)
7. Formulation by Gel filtration Sephadex G-100

### C. Manufacturing

1. Sterile filtration
2. Aseptic filling
3. Lyophilization

### TREATMENT OF OTHER PORPHYRIAS

In analogy with the new treatment of AlP patients with (recombinant) PBGD, hepatic Porphyrias such as ALA deficiency Porphyria (ADP), Porphyria cutanea tarda (PCT), Hereditary Coproporphyria (HCP) and Variegata Porphyria (VP) can benefit from substitution therapy by rALA dehydratase, rUroporphyrinogen decarboxylase, rCoproporphyrinogen oxidase and rProtoporphyrinogen oxidase, respectively.

Patients having Erythropoetic Porphyrias such as Congenital erythropoietic Porphyria (CEP) or Erythropoietic protoporphyria (EPP) will benefit from substitution therapy with rUroporphyrinogen III syntetase and rFerrochelatase, respectively.

Hepatoerythropoietic Porphyrias e.g. Hepatoerythropoietic Porphyrias(HEP) can be treated with rUroporphyrinogen decarboxylase.

All porphyrias can be treated by the administration of the enzymatic activity lacking or being reduced (normally 50%) in any of the eight steps in the heme biosynthetic pathway as described above.

The substitution of the enzymatic activity can be achieved by adding the corresponding recombinant enzyme or other molecules that will provide the missing enzymatic activity.

### GENE THERAPY AS AN ALTERNATIVE TREATMENT POSSSIBILTY FOR PATIENTS WITH ACUTE INTERMITTENT PORPHYRIA (AIP)

The human enzyme Porphobilinogen deaminase PBGD is coded for by a single gene located on chromosome 11q 24.

Mutations in this gene causes the disease Acute Intermittent Porphyria (AIP). The disease has been shown to be inhereted in an autosomal dominat way.

Today over 100 mutations in the PBGD gene has been identified (Grandchamp B. J. Ganstroenterology and Hepathology, 11, 1046-1052, 1996, Table A) and the number is expected to increase when modern diagnostic systems based on screening programs will be applied more routinely in hospitals. A number of these mutations are shown in Table A

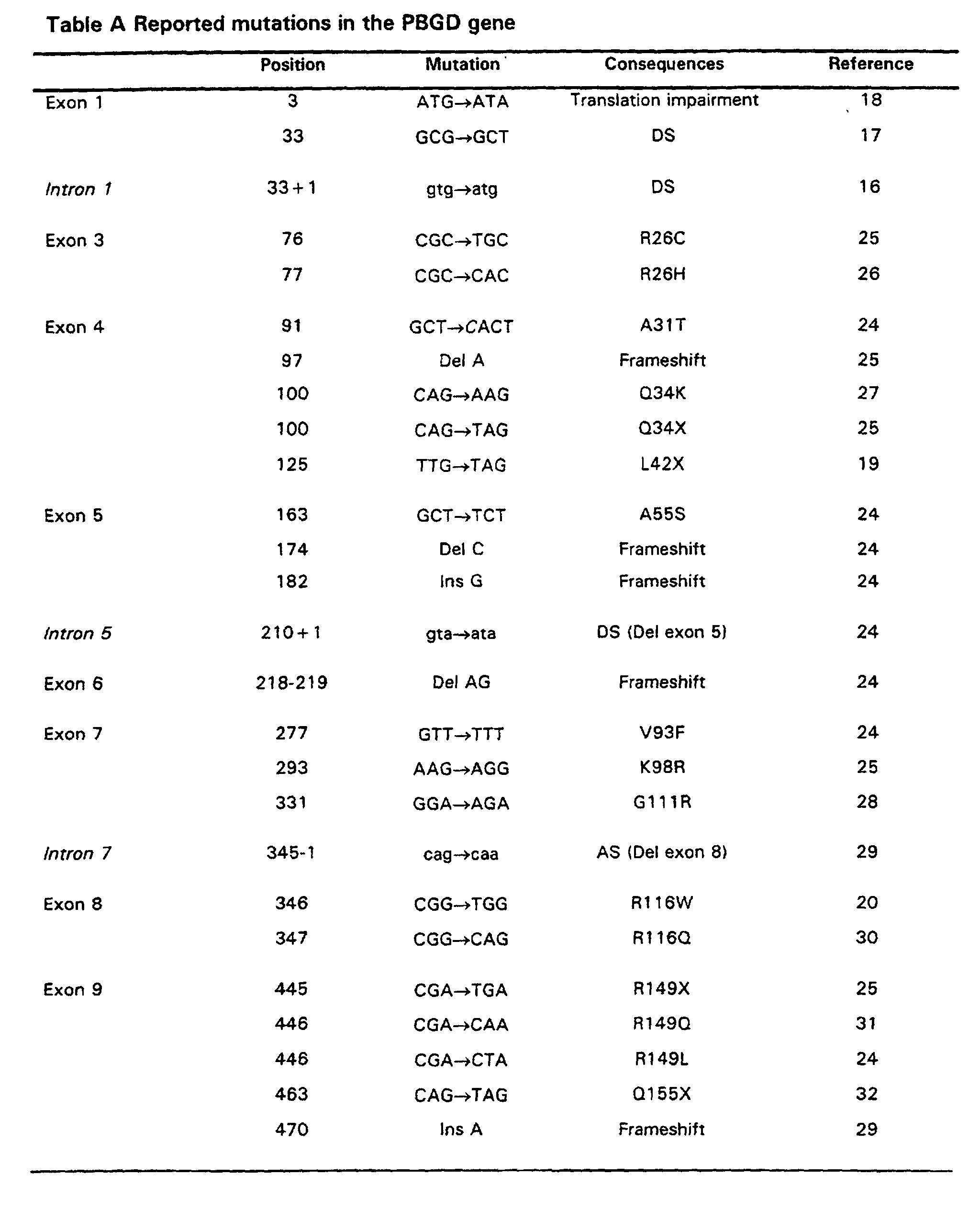

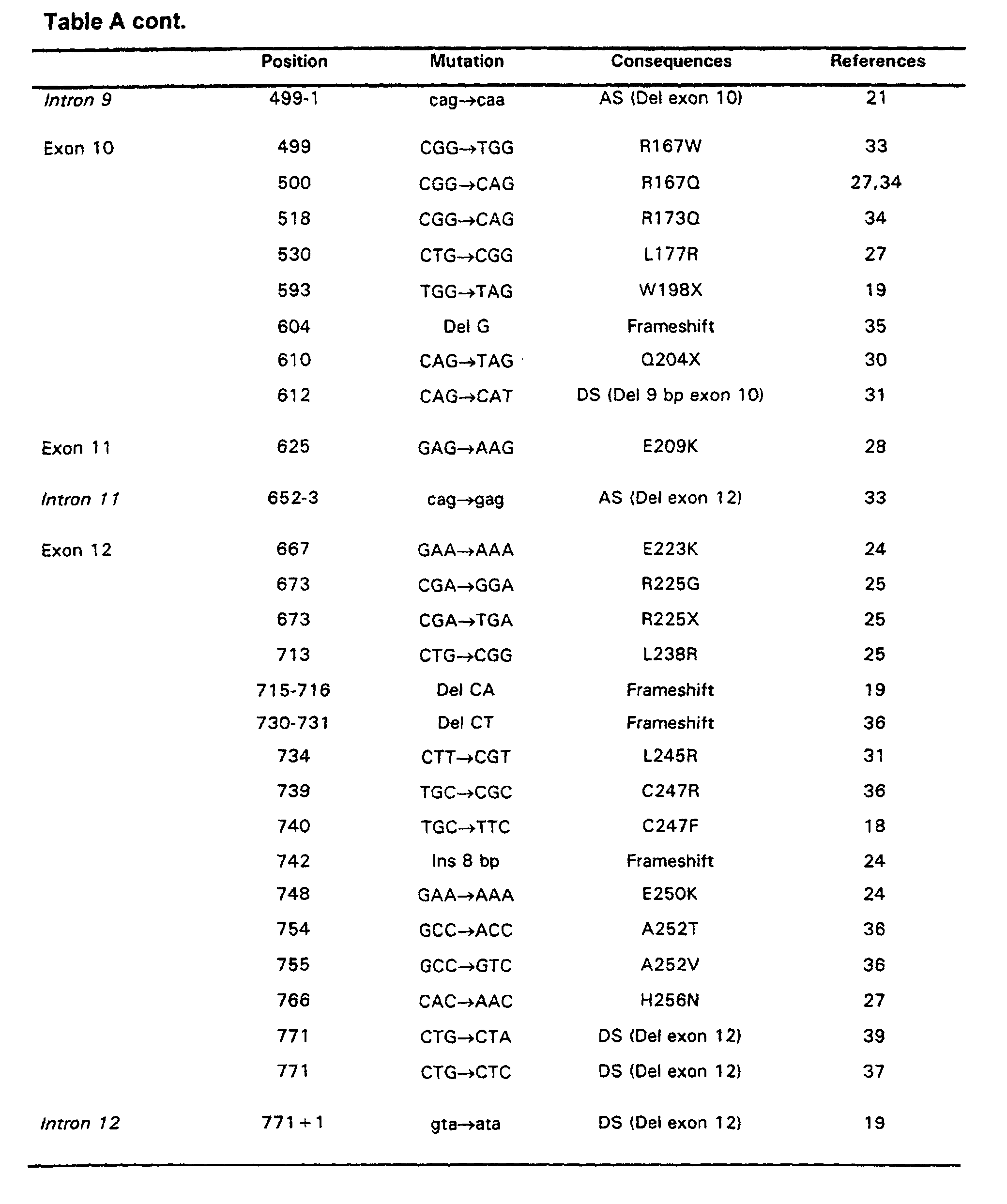

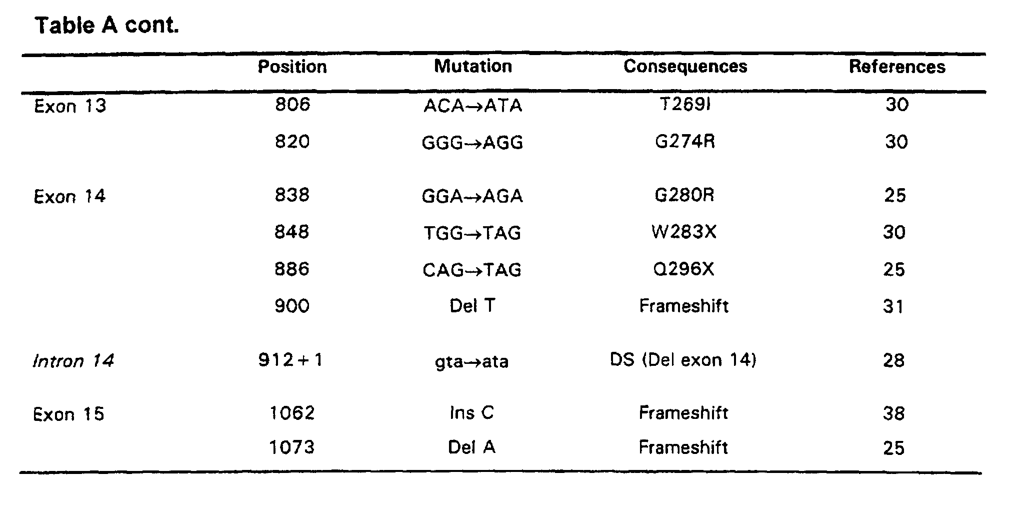

In one aspect, the present invention relates to a therapeutic method for AIP patients based on gene therapy.

The gene therapy treatment may involve the following steps.
1. Identification mutations in the PBGD gene causing AIP in humans
2. Selection of human PBGD cDNA sequence for gene therapy
3. Construction of PBGD gene therapy vectors.
4. Production of PBGD gene transfer vector
5. Delivery system of PBGD gene transfer vector

### 1. Identification of mutations in the PBGD gene causing AIP in humans

Patients having a point mutation in Exon 10 at position 593 TGG>TAG have a change in the amino acid sequence of the PBGD enzyme from W198X (stop codon). This mutation is carried by approximately 50 % of all AIP patients in Sweden (Lee JS. et al. Proc. Natl. Acad. Sci. USA, 88, 10912-10915, and 1991). AIP patients with other mutations than W198X, which might also benefit from gene therapy, are given in Table A.

### 2. Selection of human PBGD cDNA sequence for gene therapy

There are two isoenzyme forms of human PBGD e.g. erythropoietic and the non-erythropoietic form, which are formed by an alternative splicing mechanism. The non-erythropoietic form has a 17 amino acid extension on the N-terminal end of the erythropoietic PBGD form.

### Non-erythropoietic PBGD form (nPBGD):

Met-Ser-Gly-Asn-Gly-Asn-Ala-Ala-Ala-Thr-Ala-Glu-Glu-Asn-Ser-Pro-Lys-Met-Arg-Val....
ATG-TCT-GGT-AAC-GGC-ATT–GCG-GCT-GCA-ACG-GCG-GAA-GAA-AAC-AGC-CCA-AAG-ATG-
AGA-GTG..

### Erythropoietic PBGD form (ePBGD):

Met-Arg-Val-
ATG-AGA-GTG...

The nucleotide and amino acid sequence for human PBGD that will be used for gene therapy differs from that published by Raich N. et al. Nucl. Acid. Res. 14, 5955-5968, 1986 in that the amino acid residue in position **332** is an **Asn** residue rather than Thr. In order to make the "wild type enzyme" and avoiding formation of antibodies the PBGD sequence has to contain an Asn residue in position 332. The cDNA sequence that will be used for the erythropoietic PBGD form is shown above.

Patient with a defect erythropoietic PBGD enzyme will be transfected with the erythropoietic PBGD cDNA sequence and patients with a defect in the non-erythropoietic form will be transfected with the non-erythropoietic cDNA sequence.

### 3. Construction of PBGD gene therapy vectors

### Adenoviral vector system

The vector is based on adenovirus type 5 (Ad5), containing three essential genetic loci E.g. E1, E2, E4, encoding important regulatory proteins and one locus E3 which is non-essential for virus growth. Deletion of E1A and E1B region renders the virus replication deficient in vivo. Efficient complementation of the E1 function (recombinant viral stocks) can be obtained in an E1 expressing cell line such as human 293-cell line.

The human PBGD cDNA will be inserted in an adenovirus vector system.

The PBGD transgenes will be driven by the endogenous PBGD promoter or a cytolomega virus promoter (CMV).

### Retroviral vectors

Retroviral vectors are well suited for gene delivery for several reasons:
1. simplicity
2. capacity to integrate up to 8kbp DNA inserts
3. their safety, non pathogenic to humans
4. easy to improve and manipulate
5. defined integration sites of genes
6. long term regulated expression

One major disadvantage with the retroviral vectors though, is that they can only transduce dividing cells.

Most common retroviridae considered for gene therapy, are the lentiviridae and the mammalian C-type viridae. Other type retroviruses have also been considered. One such example, is a Moloney-murine leukemia retrovirus (Mo-MLV), which has been successfully used to transduce mouse and human fibroblasts with the uroporphyrinogen III synthetase (UROIIIS). (Moreau-Gaudry et al. Human Gene Therapy 6, 13-20, 1995).

The expression of the UROIIIS gene was driven by long terminal repeat (LTR). The UROIIIS cDNA was also successfully transduced by the retrovirus vectors into human peripheral blood progenitor cells.

The erythropoietic PBGD cDNA sequence can be inserted in a retrovirus vector LXSN (Miller et al BioTechniques 7, 980-990, 1989) and pMFG ( Dranoff et al. Proc. Natl. Acad.Sci. USA. 90,3539-3543, 1993). This will lead to the following constructs e.g. LePSN and pMFG-ePBGD, respectively.

### LePSN:

>      1032 bp     <
LTR----------------/ cDNA ePBGD /  SV40  /        Neo       /-------------LTR

### pMFG-ePBGD:

>     1032 bp    <
LTR--------------/  cDNA ePBGD/--------------------LTR

For transduction of non-erythropoietic tissues the non-erythropoietic cDNA (See sequence 12) will be inserted in the LSXN vector and the pMFG vector resulting in the LSnPN and pMFG-nPBGD vectors, respectively.

### LnPSN:

>     1083 bp     <
LTR---------------/ cDNA nPBGD /   SV40   /   Neo   /---------LTR

### pMFG-nPBGD:

>    1083 bp       <
LTR------------/   cDNAnPBGD/---------------LTR

The LePSN and LnPSN vectors can be converted to the corresponding virus by transfer into an appropriate host cell line e.g. Ψ CRE as described by (Danos et al. Proc. Natl. Acad. Sci. USA. 85, 6460-6464, 1988). Filtered supernatants from ectopic virus producing cells were added to amphotropic cells Ψ CRIP, in the presence of Polybrene. Clones can be isolated and tested for virus. Clones that show titers over 1.000.000 cfu/ml wilt be saved (resistant to G418).
The LnPSN vector will be cotransfected with the pMCl-Neo plasmid (Pharmacia, Sweden) into the packaging cell line Ψ CRIP. Clones that shows integration of provirus and high expression levels of message will be selected.

Filtrate from supernatants from virus producing cells (erythropoietic PBGD form) can be mixed with Polybrene and incubated with peripheral blood progenitor cells (bone marrow transplant) from an AIP patient for several hours. The transduced progenitor cells can then be transplanted back into an AIP patient.

The success of the treatment will be measured as the increase in the PBGD activity in erythrocytes and reduced excretion of ALA and PBG in the urine. Clinically a success of the treatment can be evaluated as a reduction of frequency of spontaneous acute attacks or drug-induced attacks. This will be a more convenient way of administering the recombinant PBGD enzyme than regular injections. The efficacy of the therapy can be evaluated by measuring the PBGD activity in blood and reduced excretion of PBG and ALA in the urine. Clinically, a successful treatment should result in less number of acute attacks or preferably no more attacks.

### Associated Adenovirus system (AAV)

AAV is a non-pathogenic human virus (Parvovirus) carried by more than 80% of all people. The advantage with AAV as compared to retroviral systems is that AAV can transduce both dividing and non-dividing cells. The virus genome, which is small, contains two Inverted Therminal Repeats (ITR) and a REP and CAP functions. The REP and CAP functions can be deleted and exogenous cDNA inserted.
Construction of an AAV vector containing the erythropoietic PBGD cDNA can be made. This AAV/PBGD vector will be suitable to transduce AIP patient's bone muscle cells, as a" muscle factory" for PBGD enzyme production. The PBGD cDNA will be engineered in such a way that a signal sequence for secretion will be added on the 5'-end of the cDNA. This will allow the erythropoietic PBGD enzyme to become secreted from the muscle cells into the blood stream. By this system patients will receive a constant delivery of active PBGD enzyme into the bloodstream, which will metabolize PBG thereby avoiding acute attacks.

### - Non-erythropoietic

Alternatively, liver cells can be transduced with AAV containing the non-erythropoietic PBGD cDNA. The construct will be engineered in such a way that the translated PBGD enzyme will remain intracellular e.g. contain a Met residue at the N-terminal end of the PBGD enzyme without a signal sequence for secretion in mammalian cells. The PBGD transgene will be transcribed and translated into new PBGD enzymes that will remain intracellularly. Levels of new PBGD enzymes made in the liver will normalize the PBGD activity to 100%. AIP patients have usually reduced PBGD activity (50-80%) in the liver depending on the mutation and individual variations.

This treatment would aliviate the clinical symptom e.g. acute attacks with abdominal pain and reduce excretion of PBG and ALA in the urine. The AAV containing the non-erythropoietic PBGD form can also be used to correct the genetic defect in other cell types such as neuronal tissue, pancreas spleen e.g. non-erythropoietic tissue, by a similar mechanism.

### - Erythropoietic

The erythropoietic PBGD cDNA can be inserted in an AAV vector and used to transduce erythropoietic cells and stem cells in AIP patients, having a mutation affecting the erythropoietic form of PBGD.

### 4. Production of PBGD gene transfer vector

Adenovirus have approximately 36 kbp double stranded DNA, containing three essential early gene loci (E1, E2, and E4) encoding important regulatory proteins. Loci E3 codes for a gene product that block immune response to virus infected cells *in vivo.* The PBGD gene transfer adenovirus vector can be produced by deleting the E1 and E3 loci. The PBGD gene cassette is inserted in that position instead. The virus will be replication defective when the E1 locus has been deleted. Efficient E1 complementation and thus high yield of recombinant virus vector (PBGD) can be obtained in an E1 expressing cell line, such as the human 293 cell line. (Graham, F. et al. 1977, Characteristics of a human cell line transformed by DNA from human adenovirus 5. J. Gen. Virol. 36, 59-72).

### 5. Delivery systems of PBGD gene transfer vectors.

Delivery of viral vectors are based on injection into the patient of a virus particle that will transduce human cells in vivo.

### Correction of point mutations causing AIP by Chimeraplasty Gene Repair

The basic technique involves the synthesis of chimeric (RNA-DNA) oligonucleotides. The oligonucleotide will repair point mutations on the chromosome by binding to the site of mutation and create a mismatch. The endogenous "mismatch repair system" which is present in all living cells, will correct the mutation.

The Chimeric oligonucleotides has the following general properties:
a. 68 mer (65-70 is acceptable size)
b. 25 base DNA stretches at the 5'-end homologous to the normal sequence of the gene
c. the 25 base DNA is designed in such a way the 12 bp on each side of the mutation is complementary to "wild-type DNA" where the mutation to be altered is located at position 13
d. the 25 mere contains 4 T bases at the one end to loop back the oligo to the other DNA strand with a 25 base sequence homologous to the other strand of the chromosomal DNA.
e. the second strand is chimeric in that it contains 10 homologous bases of 2'O methyl RNA followed by 5 bases of DNA (containing a central mismatch e.g. correction of the human point mutation by mismatch repair) followed by another stretch of 10 bases of homologous 2'O methyl RNA. This stretch of DNA/RNA is followed with 5 bases of GC clamp and 4 T bases to form the second loop and finally a 5 base CG clamps complementary to the other one.

### EXAMPLE 1

### Correction of the PBGD mutation at position 593 TGG>TAG resulting in W198X

### Normal Chromosomal Sequence:

5´-AG CGC ATG GGC TGG CAC AAC CGG GT-3´
Gln Arg Met Gly Trp His Asn Arg Val

### AIP Chromosomal Sequence:

5´- AG CGC ATG GGC TAG CAC AAC CGG GT-3´
Stop

### The sequence of the chimeric oligonucleotide (Heme593W/X) is:

DNA
G
RNA ↓ ⇓ ↓ RNA
Chromosome ..... 5'- AG CGC ATG GGC TAG CAC AAC CGG GT-3'.................
gggccTC GCG TAC CCG ACC GTG TTG GCC CA
T T
( )
loop T Heme593W/X T loop
T T
( )
Tcccgg T
5'-AG CGC ATG GGC TGG CAC AAC CGG GT
Chromosome ..... 3'-TC GCG TAC CCG ATC GTG TTG GCC CA -5'...............
⇑
C

The same principle of chimeric oligonucleotide can be constructed to correct any of the mutations causing AIP depicted in Table A.

Chimeric oligonucleotides can be used to correct any other point mutation causing any of the 8 known Porphyrias in a similar way as described above.

### Delivery of PBGD gene transfer of non viral vectors to humans

The chimeric oligonucleotide can be formulated in a vechicles preparation containing anionic or cationic phospholipids or phospholipids mixed with neutral lipids or lictosylated PEI.

Alternatively, the non-viral vectors can be formulated in liposomes containing mixtures of natural phospholipids and neutral lipids.

Specific protein sequences can be incorporated into liposomal membranes, that recognizes cellular receptors for specific targeting of non-viral vectors to a specific cell type such as liver, neuronal tissue or erythropoietic tissues, can be incorporated. Alternatively specific antibodies recognizing specific cellular surface antigens can be used for targeting. Thirdly, carbohydrates on the liposomal membrane can be used for liver uptake of chimeric oligonucleotides.

The formulated chimeric oligonucleotide (HemeBiotech 595 W/X) will be administered by sc. or IV. injections to AIP patients.

The efficacy of the treatment can be evaluated as above.

### GENE THERAPY AS AN ALTERNATIVE TREATMENT OF OTHER PORPHYRIC DISEASES

The gene therapy strategies outlined herein can also be used for other Porphyric diseases. The general principle is to increase the cellular or systemic content of a particular defective enzyme causing the disease. The following Porphyric diseases can be encompassed by this strategy:
1. ALA deficiency porphyria (ADP)
2. Porphyria cutanea tarda (PCT)
3. Hereditary coproporphyria (HCP)
4. Harderoporphyria (HDP)
5. Variegata porphyria (VP)
6. Congenital erythropoietic porphyria (CEP)
7. Erythropoietic protoporphyria (EPP)
8. Hepatoerythropoietic porphyria (HEP)

### References:

Andersson, Christer, Thesis, 1997, ISBN 91/7191/280/0, pp. 22-23

Danos et al. Proc. Natl. Acad. Sci. USA. 85, 6460-6464, 1988

Dranoff et al. Proc. Natl. Acad.Sci. USA. 90,3539-3543, 1993

Grandchamp B, et al Tissue-specific expression of porphobilinogen deaminase. Two isoenzymes from a single gene. Eur J Biochem. 1987 Jan;162(1):105-10.

Grandschamp B. et al. 1996. J. of Gastroenerology and Hepatology 11, 1046-1052

Herrick A.L. et al 1989, Lancet 1, 1295-1297

Jeans J. et al. American J. of Medical Genetics 1996,65, 269-273

Lithner F. et al. 1984, Acta.Med.Scand. 215,271-274

Maniatis T., E.F. Fritsch, J. Sambrook. Molecular Cloning (A laboratory Manual) Cold Spring Harbor Laboratory. 1982.

Miller et al BioTechniques 7, 980-990, 1989

Moreau-Gaudry et al. Human Gene Therapy 6, 13-20,1995).

Mustajoki et al. 1989, Sem. Hematol. 26, 1-9).

Raich N, et al Molecular cloning and complete primary sequence of erythrocyte porphobilinogen deaminase. Nucleic Acids Research 1986 14(15): 5955-67

Raich N. et al 1986, Nucleic. Acid. Res, 14, 5955-5968

Saiki RK, et al Enzymatic amplification of beta-globin genomic sequences and restriction site analysis for diagnosis of sickle cell anemia. Science 1985 Dec 20;230(4732):1350-4.

Sassa S. 1996, Blood Review, 10, 53-58

Stephens P.E. et. al. Biochem J. 248, 1-11, 1987

Strand et al. Proc. Natl. Acad. Sci. 1970, 67, 1315-1320

Tishler P.V: et al. 1985, Am.J.Psychiatry 142,1430-1436

Waldenström J. Acta.Med. Scand. 1937 Suppl.82

Welland F.H. et al. 1964, Metabolism,13, 232

Wetterberg L. 1967, Svenska bokförlaget Nordstedt,Stockholm

Wetterberg L. 1976, In Doss M. Nowrocki P. eds. Porphyrias in Human Disease.

Reports of the discussion. Matgurg an der Lahn, 191-202

Yeung L. et al. 1983, Q J. Med 52, 92-98

Yoo HW, et. Al. Hydroxymethylbilane synthase: complete genomic sequence and amplifiable polymorphisms in the human gene. Genomics 1993 Jan; 15(1):21-9.

## Claims

1. Use of an effective amount of a catalyst which is an enzyme belonging to the heme biosynthetic pathway or an enzymatically equivalent part or analogue thereof together with a pharmaceutically acceptable carrier for the preparation of a pharmaceutical composition for treatment or prophylaxis of a disease caused by a deficiency, in a subject, of an enzyme belonging to the heme biosynthetic pathway.

2. Use according to claim 1, wherein the disease is selected from the group consisting of
acute intermittent porphyria (AIP),
ALA deficiency porphyria (ADP),
Porphyria cutanea tarda (PCT),
Hereditary coproporphyria (HCP),
Harderoporphyria (HDP),
Variegata porphyria (VP),
Congenital erythropoetic porphyria (CEP),
Erythropoietic protoporphyria (EPP), and
Hepatoerythropoietic porphyria (HEP).

3. Use according to claim 1 or 2, wherein the catalyst is an enzyme selected from the group consisting of
porphobilinogen deaminase (PBGD)
ALA dehydratase,
Uroporphyrinogendecarboxylase,
Coproporphyrinogen oxidase,
Coproporphyrinogen oxidise,
Protoporphyrinogen oxidase,
Uroporphyrinogen III synthase,
Ferrochelatase, and
Uroporphyrinogen decarboxylase,
or an enzymatically equivalent part or analogue thereof.

4. Use according to any of the preceding claims, wherein the disease is AIP and the enzyme is PBGD or an enzymatically equivalent part or analogue thereof.

5. Use according to any of the preceding claims, wherein the catalyst is a recombinant form of the enzyme belonging to the heme biosynthetic pathway or of the enzymatically equivalent part or analogue thereof.

6. Use according to any of the preceding claims, wherein the catalyst is administrable by a route selected from the group consisting of the intravenous route, the intraarterial route, the intracutaneous route, the subcutaneous route, the oral route, the buccal route, the intramuscular route, the anal route, the transdermic route, the intradermal route, and the intratechal route.

7. Use according to any of claims 1-6, wherein the catalyst is formulated in an isotonic solution, such as 0.9% NaCl and 10-50 mM Sodium phosphate pH 6.50 to 8 or Sodium phosphate, glycine, mannitol or the corresponding potassium salts.

8. Use according to any of claims 1-7, wherein the catalyst is lyophilised.

9. Use according to any of claims 1-8, wherein the catalyst is sterile filtered.

10. Use according to any of claims 1-6, 8 or 9, wherein the catalyst is formulated as lipid vesicles comprising phosphatidylcholine or phosphatidylethanolamine or combinations thereof.

11. Use according to any of claims 1-6, 8 or 9, wherein the catalyst is incorporated into erythrocyte ghosts.

12. Use according to any of claims 1-6, 8 or 9, wherein the catalyst is formulated as a sustained release formulation involving biodegradable microspheres, such as microspheres comprising polylactic acid, polyglycolic acid or mixtures of these.

13. Use according to any of claims 1-9, wherein the catalyst is lyophilized in a two-compartment cartridge, where the catalyst will be in the front compartment and water for reconstitution in the rear compartment.

14. Use according to claim 13, wherein the two compartment cartridge is combined with an injection device to administer the catalyst either by a needle or by a needle-less (high pressure) device.

15. Use according to any of claims 1-9, wherein the catalyst is formulated in a physiological buffer containing an enhancer for nasal administration.

16. Use according to any of the preceding claims, wherein the catalyst is formulated as an oral formulation containing lipid vesicles, such as those comprising phospatidylcholine, phosphatidylethanolamine, or sphingomyeline, or dextrane microspheres.

17. Use according to any of the preceding claims, wherein the catalyst is formulated so as to enhance the half-life thereof in the subject's bloodstream.

18. Use according to claim 17, wherein the catalyst has a polyethylene glycol coating.

19. Use according to claim 17, wherein the catalyst is complexed with a heavy metal.

20. Use according to any of the preceding claims, wherein the catalyst is an enzymatically equivalent part or analogue of the enzyme and exerts at least part of its enzymatic activity intracellularly upon administration to the subject.

21. Use according to claim 20, wherein the catalyst is a small artificial enzyme or an organic catalyst which can polymerize porphobilinogen to hydroxymethylbilane.

22. Use according to any of claims 1-9, wherein the catalyst is said enzyme formulated in such a manner that it exerts at least part of its enzymatic activity intracellularly upon administration to the subject.

23. Use according to claim 22, wherein the catalyst is tagged with specific carbohydrates or other liver cell specific structures for specific fiver uptake.

24. Use according to claims 1-19, wherein the catalyst exerts substantially all its enzymatic activity extracellularly in the bloodstream.

25. Use according to claim 24, wherein the enzymatic activity of the catalyst on its relevant heme precursor results in a metabolic product which 1) either moves into the intracellular compartment and is converted further via the remaining steps of the heme biosynthetic pathway or 2) is excreted from the subject via urine and/or faeces.

26. Use according to any of the preceding claims, wherein the catalyst has been prepared by a process comprising
a) introducing, into a suitable vector, a nucleic acid fragment which includes a nucleic acid sequence encoding the catalyst;
b) transforming a compatible host cell with the vector;
c) culturing the transformed host cell under conditions facilitating expression of the nucleic acid sequence; and
d) recovering the expression product from the culture
and optionally subjecting the expression product to post-translational processing, such as in vitro protein refolding, enzymatic removal of fusion partners, alkylation of amino acid residues, and deglycosylation, so as to obtain the catalyst.

27. Use according to any of claims 1-25, wherein the catalyst has been prepared by liquid-phase or solid-phase peptide synthesis.

28. Use according to any of the preceding claims, wherein the catalyst is free from any other biological material of human origin.

29. Use according to any of the preceding claims, wherein the catalyst is administrable at least once a day, such as 2, 3, 4, and 5 times daily.

30. Use according to any of the preceding claims wherein the daily dosage is in the range of 0.01 - 1.0 mg/kg body weight per day, such as in the range of 0.05 - 0.5 mg/kg body weight per day.

31. Use according to any of the preceding claims, wherein the daily dosage is about 0.1 mg per kg body weight per day.

32. Use according to any of the preceding claims, wherein the composition is adapted for administration at least once a day, such as 2, 3, 4, and 5 times daily and wherein the daily dosage is about 0.1 mg per kg body weight per day.

33. Use according to any of claims 1-31 wherein the catalyst is a recombinant form of the enzyme.

34. Use according to any of claims 1-33 wherein the catalyst is recombinant human PBGD based on any of Seq. ID NO 1 (clone PBGD 1.1) and Seq. ID NO 12 (non-erythro PBGD 1.1.1).

35. A catalyst which is an enzyme of the heme biosynthetic pathway or an enzymatically equivalent part or analogue thereof, for use as a medicament.

36. A catalyst according to claim 35, which is recombinant human PBGD based on any of Seq. ID NO 1 (clone PBGD 1.1) and Seq. ID NO 12 (non-erythro PBGD 1.1.1).

37. Use of a human PBGD cDNA sequence of either non-erythropoietic form or erythropoietic form for the preparation of a medicament for gene therapy by transfection of the patient with the human PBGD cDNA sequence of either non-erythropoietic form or erythropoietic form according to the tissue in which PBGD should be expressed, for treatment or preventing disease in a patient having a mutation in the PBGD gene causing an enzyme defect.

38. Use according to claim 37 wherein the enzyme defect is selected from enzyme defiencies resulting in a disease selected from Acute Intermittent Porphyria (AIP), ALA deficiency porphyria (ADP), Porphyria cutanea tarda (PCT), Hereditary coproporphyria (HCP), Harderoporphyria (HDP), Variegata porphyria (VP), Congenital erythropoietic porphyria (CEP), Erythropoietic protoporphyria (EPP), and Hepatoerythropoietic porphyria (HEP).

39. Use according to claim 37 wherein the disease is Acute Intermittent Porphyria, (AIP).

40. Use according to any of claims 37-39 wherein the human PBGD cDNA sequence is selected from Seq. ID NO 1 (clone PBGD 1.1) and Seq. ID NO 12 (non-erythro PBGD 1.1.1)

41. Use according to any of claims 37-40 wherein the transfection is by use of a vector selected from adenovirus, retrovirus and associated adenovirus.

42. Use according to any of claims 37-41 wherein the PBGD gene transfer vector into human cells (erythropoeitic and/or non-erythropoietic) results in normal PBGD activity.

43. Use of a specific designed oligonucleotide molecule for the manufacture of a medicament for the gene therapy treatment of patients with Acute Intermittent Porphyria (AIP) by a correction of one of the specific point mutations identified causing AIP by use of chimeraplasty gene repair.

44. Use according to any of claims 37-43 wherein the delivery system for transfection is by use of non-viral vectors formulated in a vehicle preparation comprising one or more components selected from cationic phospholipids, phospholipids, phospholipids mixed with neutral lipids, lictosylated PEI, liposomes comprising mixtures of natural phospholipids and neutral lipids.

45. Use according to claim 43 or 44 wherein the mutation is selected from the following Table A :

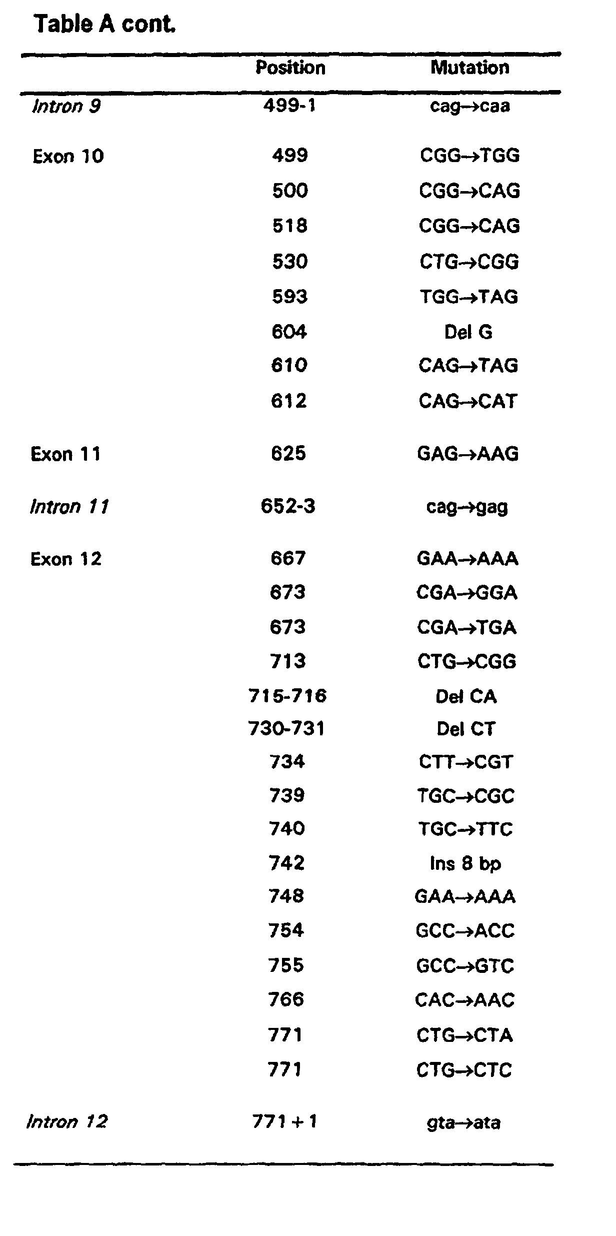

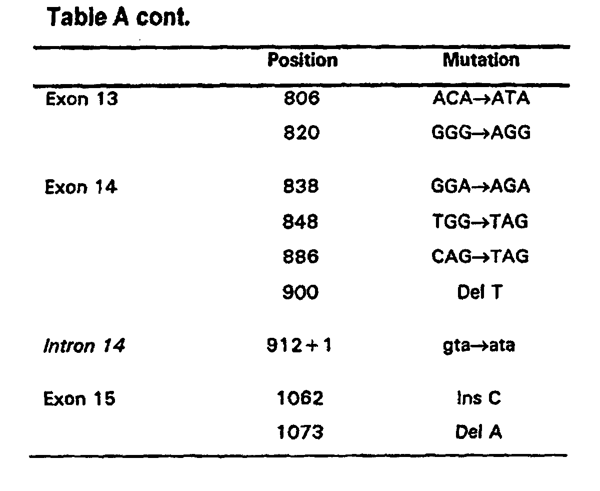

## Patentansprüche

1. Verwendung einer wirksamen Menge eines Katalysators, welcher ein zum Häm-Biosyntheseweg gehörendes Enzym oder ein enzymatisch äquivalenter Teil oder ein Analoges davon ist, und einen pharmazeutisch verträglichen Träger für die Herstellung eines Arzneimittels zur Behandlung oder Prophylaxe einer Krankheit, verursacht durch den Mangel eines Enzyms, das zum Häm-Biosyntheseweg gehört, in einem Patienten.

2. Verwendung nach Anspruch 1, wobei die Krankheit ausgewählt ist aus der Gruppe bestehend aus
akuter intermittierender Porphyrie (AIP),
ALA-Mangel-Porphyrie (ADP),
Porphyria cutanea tarda (PCT),
erblicher Coproporphyrie (HCP),
Harderoporphyrie (HDP),
Variegata porphyria (VP),
congenitaler erythropoetischer Porphyrie (CEP),
erythropoetischer Protoporphyrie (EPP), und
hepatoerythropoetischer Porphyrie (HEP).

3. Verwendung nach Anspruch 1 oder 2, wobei der Katalysator ein Enzym ist, ausgewählt aus der Gruppe bestehend aus
Porphobilinogen-Deaminase (PBGD),
ALA-Dehydratase,
Uroporphyrinogen-Decarboxylase,
Coproporphyrinogen-Oxidase,
Coproporphyrinogen-Oxidise,
Protoporphyrinogen-Oxidase,
Uroporphyrinogen-III-Synthase,
Ferrochelatase, und
Uroporphyrinogen-Decarboxylase,
oder ein enzymatisch äquivalenter Teil oder Analoges davon.

4. Verwendung nach einem der vorangehenden Ansprüche, wobei die Krankheit AIP ist, und das Enzym PBGD oder ein enzymatisch äquivalenter Teil oder Analoges davon ist.

5. Verwendung nach einem der vorangehenden Ansprüche, wobei der Katalysator eine rekombinante Form des zum Häm-Biosyntheseweg gehörenden Enzyms oder des enzymatisch äquivalenten Teils oder Analogen davon ist.

6. Verwendung nach einem der vorangehenden Ansprüche, wobei der Katalysator über einen Weg verabreichbar ist, der ausgewählt ist aus der Gruppe bestehend aus dem intravenösen Weg, dem intraarteriellen Weg, dem intrakutanen Weg, dem subkutanen Weg, dem oralen Weg, dem bukkalen Weg, dem intramuskulären Weg, dem analen Weg, dem transkutanen Weg, dem intradermalen Weg und dem intrathecalen Weg.

7. Verwendung nach einem der Ansprüche 1 bis 6, wobei der Katalysator in einer isotonischen Lösung formuliert ist, wie 0,9% NaCl und 10-50 mM Natriumphosphat pH 6,50 bis 8 oder Natriumphosphat, Glycin, Mannit oder die entsprechenden Kaliumsalze.

8. Verwendung nach einem der Ansprüche 1 bis 7, wobei der Katalysator lyophilisiert ist.

9. Verwendung nach einem der Ansprüche 1 bis 8, wobei der Katalysator sterilfiltriert ist.

10. Verwendung nach einem der Ansprüche 1 bis 6, 8 oder 9, wobei der Katalysator als Lipidvesikel formuliert ist, die Phosphadtidylcholin oder Phosphatidylethanolamin oder Kombinationen davon umfassen.

11. Verwendung nach einem der Ansprüche 1 bis 6, 8 oder 9, wobei der Katalysator in Erythrozytenghosts eingebaut ist.

12. Verwendung nach einem der Ansprüche 1 bis 6, 8 oder 9, wobei der Katalysator als Formulierung mit verzögerter Freisetzung formuliert ist, die biologisch abbaubare Mikrosphären einschließt, wie Mikrosphären umfassend Polymilchsäure, Polyglykolsäure oder Gemische aus diesen.

13. Verwendung nach einem der Ansprüche 1 bis 9, wobei der Katalysator in einer Patrone aus zwei Kompartimenten lyophilisiert ist, worin der Katalysator im vorderen Kompartiment ist und Wasser zur Wiederherstellung im hinteren Kompartiment.

14. Verwendung nach Anspruch 13, wobei die aus zwei Kompartimenten bestehende Patrone mit einer Injektionsvorrichtung zur Verabreichung des Katalysators entweder durch eine Nadel oder durch eine nadellose (Hochdruck-) Vorrichtung kombiniert ist.

15. Verwendung nach einem der Ansprüche 1 bis 9, wobei der Katalysator in einem physiologischen Puffer formuliert ist, der einen Verstärker zur nasalen Verabreichung enthält.

16. Verwendung nach einem der vorangehenden Ansprüche, wobei der Katalysator als orale Formulierung formuliert ist, die Lipidvesikel enthält, wie solche, die Phosphatidylcholin, Phosphatidylethanolamin oder Sphingomyelin umfassen, oder Dextranmikrosphären.

17. Verwendung nach einem der vorangehenden Ansprüche, wobei der Katalysator in einer Weise formuliert ist, dass seine Halbwertszeit im Blutstrom des Patienten verlängert ist.

18. Verwendung nach Anspruch 17, wobei der Katalysator eine Polyethylenglykol-Hülle hat.

19. Verwendung nach Anspruch 17, wobei der Katalysator mit einem Schwermetall komplexiert ist.

20. Verwendung nach einem der vorangehenden Ansprüche, wobei der Katalysator ein enzymatisch äquivalenter Teil oder Analoges des Enzyms ist und bei Verabreichung an den Patienten intrazellulär mindestens einen Teil seiner enzymatischen Aktivität ausübt.

21. Verwendung nach Anspruch 20, wobei der Katalysator ein kleines künstliches Enzym oder ein organischer Katalysator ist, der Porphobilinogen zu Hydromethylbilan polymerisieren kann.

22. Verwendung nach einem der Ansprüche 1 bis 9, wobei der Katalysator das Enzym ist, welches in einer Weise formuliert ist, daß es bei Verabreichung an den Patienten intrazellulär mindestens einen Teil seiner enzymatischen Aktivität ausübt.

23. Verwendung nach Anspruch 22, wobei der Katalysator mit spezifischen Kohlenhydraten oder anderen leberzellspezifischen Strukturen zur spezifischen Leberaufnahme markiert ist.

24. Verwendung nach einem der Ansprüche 1 bis 19, wobei der Katalysator im wesentlichen seine gesamte enzymatische Aktivität extrazellulär im Blutstrom ausübt.

25. Verwendung nach Anspruch 24, wobei die enzymatische Aktivität des Katalysators bezüglich seiner relevanten Hämvorstufe zu einem Stoffwechselprodukt führt, welches
(1) sich entweder in das intrazelluläre Kompartiment bewegt und mittels der verbliebenen Schritte des Häm-Biosynthesewegs weiter umgesetzt wird, oder
(2) vom Patienten via Urin und/oder Faeces ausgeschieden wird.

26. Verwendung nach einem der vorangehenden Ansprüche, wobei der Katalysator durch ein Verfahren hergestellt wird, umfassend
(a) Einführen eines Nucleinsäurefragmentes, das eine den Katalysator codierende Nucleinsäuresequenz enthält, in einen geeigneten Vektor;
(b) Transformieren einer kompatiblen Wirtszelle mit dem Vektor;
(c) Züchten der transformierten Wirtszelle unter Bedingungen, die die Expression der Nucleinsäuresequenz fördern; und
(d) Gewinnung des Expressionsproduktes aus der Kultur, und gegebenenfalls Durchführen einer posttranslationalen Prozessierung am Expressionsprodukt, wie Rückfaltung des Proteins in vitro, enzymatische Entfernung von Fusionspartnern, Alkylierung von Aminosäureresten und Deglykosylierung, um den Katalysator zu erhalten.

27. Verwendung nach einem der Ansprüche 1 bis 25, wobei der Katalysator durch Flüssigphasen- oder Festphasenpeptidsynthese hergestellt wird.

28. Verwendung nach einem der vorangehenden Ansprüche, wobei der Katalysator frei von jeglichem anderen biologischen Material menschlichen Ursprungs ist.

29. Verwendung nach einem der vorangehenden Ansprüche, wobei der Katalysator mindestens einmal täglich verabreichbar ist, wie zwei-, drei-, vierund fünfmal täglich.

30. Verwendung nach einem der vorangehenden Ansprüche, wobei die tägliche Dosis im Bereich von 0,01 bis 1,0 mg/kg Körpergewicht pro Tag, wie im Bereich von 0,05 bis 0,5 mg/kg Körpergewicht pro Tag ist.

31. Verwendung nach einem der vorangehenden Ansprüche, wobei die tägliche Dosis etwa 0,1 mg/kg Körpergewicht pro Tag ist.

32. Verwendung nach einem der vorangehenden Ansprüche, wobei die Zusammensetzung für eine mindestens einmalige Verabreichung pro Tag, wie zwei-, drei-, vier-, und fünfmal täglich, angepasst ist, und wobei die tägliche Dosis etwas 0,1 mg/kg Körpergewicht pro Tag ist.

33. Verwendung nach einem der Ansprüche 1 bis 31, wobei der Katalysator eine rekombinante Form des Enzyms ist.

34. Verwendung nach einem der Ansprüche 1 bis 33, wobei der Katalysator rekombinantes, menschliches PBGD, basierend auf einer der SEQ ID NO: 1 (Clon PBGD 1.1) und SEQ ID NO: 12 (Nicht-erythro PBGD 1.1.1), ist.

35. Katalysator, welcher ein Enzym des Häm-Biosynthesewegs oder ein enzymatisch äquivalenter Teil oder Analoges davon ist, zur Verwendung als Medikament.

36. Katalysator nach Anspruch 35, welcher rekombinantes, menschliches PBGD basierend auf einer der SEQ ID NO: 1 (Clon PBGD 1.1) und SEQ ID NO: 12 (Nicht-erythro PBGD 1.1.1) ist.

37. Verwendung einer menschlichen PBGD-cDNA-Sequenz, entweder der nichterythropoetischen Form oder der erythropoetischen Form, für die Herstellung eines Medikamentes zur Gentherapie durch Transfektion eines Patienten mit der menschlichen PBGD-cDNA-Sequenz, entweder der nichterythropoetischen Form oder der erythropoetischen Form entsprechend dem Gewebe, in welchem PBGD exprimiert werden soll, zur Behandlung oder Verhinderung einer Krankheit in einem Patienten, der eine Mutation im PBGD-Gen hat, die einen Enzymdefekt verursacht.

38. Verwendung nach Anspruch 37, wobei der Enzymdefekt ausgewählt ist aus Enzymmängeln, die zu einer Krankheit führen, ausgewählt aus
akuter intermittierender Porphyrie (AIP),
ALA-Mangel-Porphyrie (ADP),
Porphyria cutanea tarda (PCT),
erblicher Coproporphyrie (HCP),
Harderoporphyrie (HDP),
Variegata porphyria (VP),
congenitaler erythropoetischer Porphyrie (CEP),
erythrophoetischer Protoporphyrie (EPP), und
hepatoerythropoetischer Porphyrie (HEP).

39. Verwendung nach Anspruch 37, wobei die Krankheit akute intermittierende Porphyrie (AIP) ist.

40. Verwendung nach einem der Ansprüche 37 bis 39, wobei die menschliche PBGD-cDNA-Sequenz ausgewählt ist aus SEQ ID NO: 1 (Clon PBGD 1.1) und SEQ ID NO: 12 (Nicht-erythro-PBGD 1.1.1).

41. Verwendung nach einem der Ansprüche 37 bis 40, wobei die Transfektion unter Verwendung eines Vektors erfolgt, ausgewählt aus Adenovirus, Retrovirus und assoziiertem Adenovirus.

42. Verwendung nach einem der Ansprüche 37 bis 41, wobei der PBGD-Gentransfer in menschliche Zellen (erythropoetisch und/oder nichterythropoetisch) zu normaler PBGD-Aktivität führt.

43. Verwendung eines spezifischen Oligonucleotidmoleküls für die Herstellung eines Medikaments zur Gentherapiebehandlung vom Patienten mit akuter intermittierender Porphyrie (AIP) durch die Korrektur einer der spezifischen Punktmutationen, die als Verursacher von AIP identifiziert sind, durch die Verwendung einer Chimäraplastie-Genreparatur.

44. Verwendung nach einem der Ansprüche 37 bis 43, wobei das Verarbreichungssystem für die Transfektion die Verwendung von nicht-viralen Vektoren umfasst, die in einem Trägerpräparat formuliert sind, umfassend eine oder mehrere Komponenten ausgewählt aus kationischen Phospholipiden, Phospholipiden, Phospholipiden gemischt mit neutralen Lipiden, liktosylierten PEI, Liposomen, umfassend Gemische aus natürlichen Phospholipiden und neutralen Lipiden.

45. Verwendung nach Anspruch 43 oder 44, wobei die Mutation aus der folgenden Tabelle A ausgewählt ist:

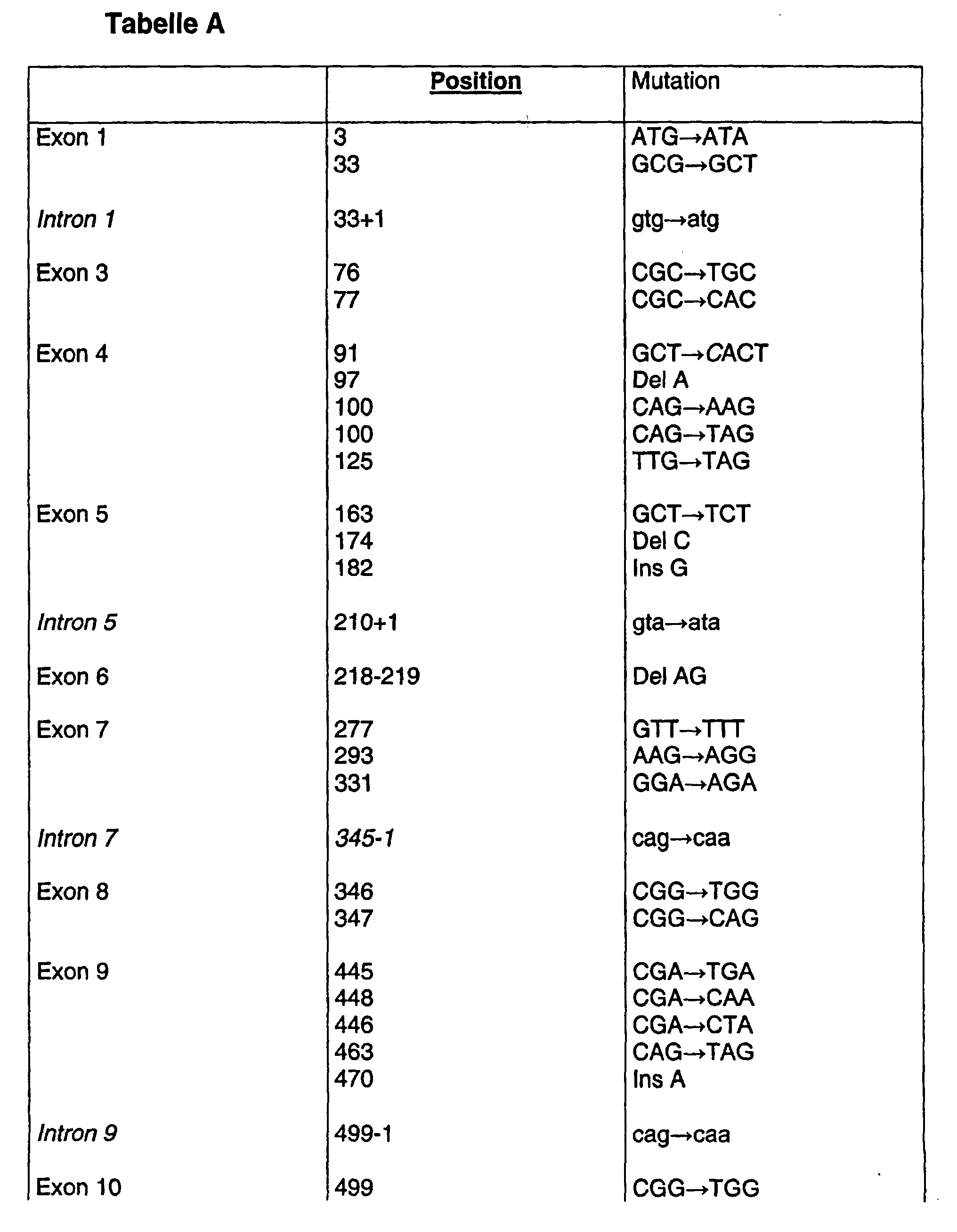

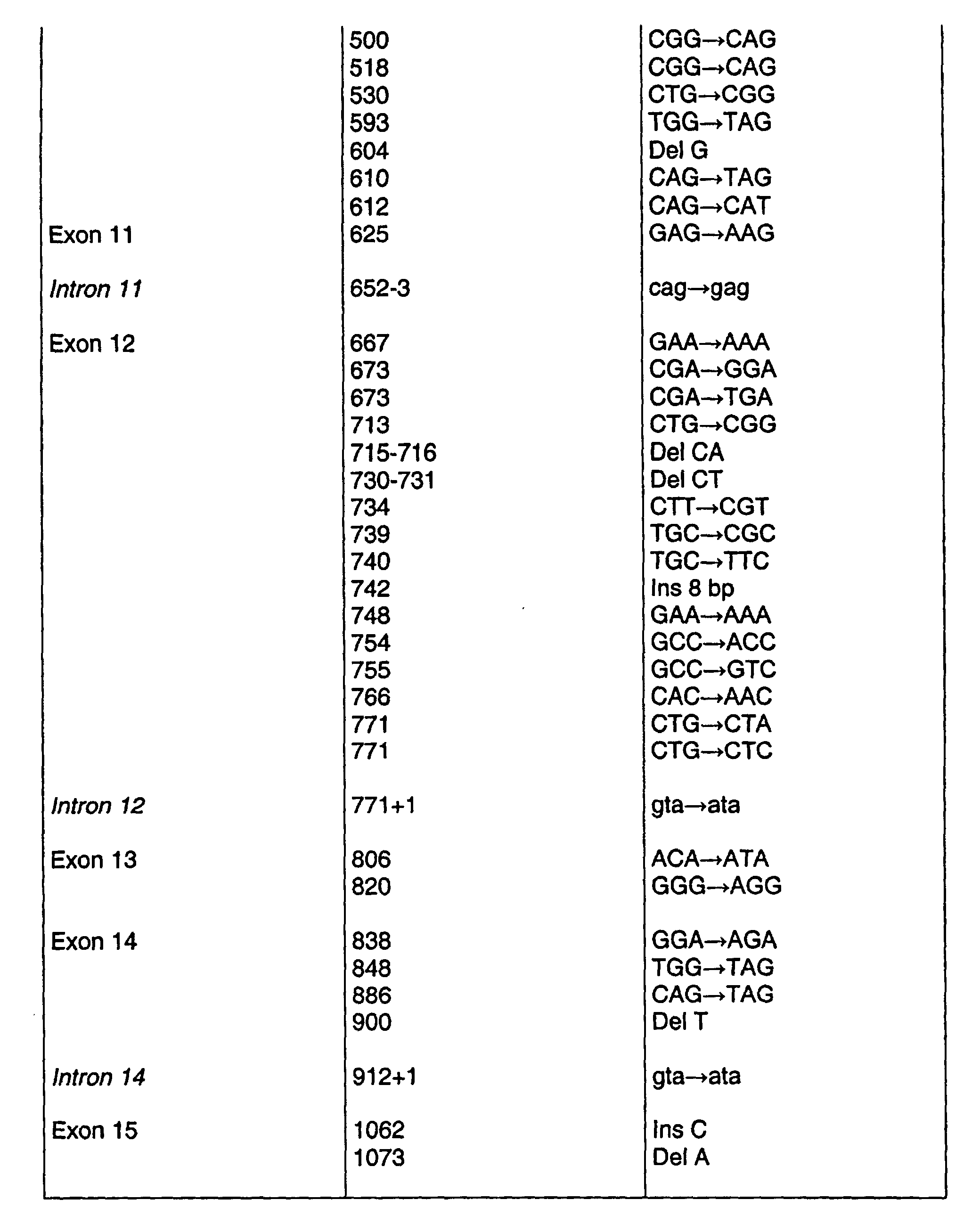

## Revendications

1. Utilisation d'une quantité efficace d'un catalyseur qui est une enzyme appartenant à la voie de biosynthèse de l'hème ou une partie équivalente sur le plan enzymatique ou un analogue de celle-ci avec un support pharmaceutiquement acceptable pour la préparation d'une composition pharmaceutique destinée au traitement ou à la prophylaxie d'une maladie causée par une carence, chez un sujet, en une enzyme appartenant à la voie de biosynthèse de l'hème.

2. Utilisation selon la revendication 1, où la maladie est choisie parmi le groupe constitué par:
la porphyrie aiguë intermittente (PAI),
la porphyrie à carence en ALA (PDA),
la porphyrie cutanée tardive (PCT),
la coproporphyrie héréditaire (CPH),
l'hardéroporphyrie (HDP),
la porphyrie variegata (PV),
la porphyrie érythropoiëtique congénitale (PEC),
la protoporphyrie érythropoiétique (PEP), et
la porphyrie hépatoérythropoiëtique (PHP).

3. Utilisation selon la revendication 1 ou la revendication 2, où le catalyseur est une enzyme choisie parmi le groupe constitué par :
la porphobilinogène désaminase (PBGD)
l'ALA déshydrase,
l'uroporphyrinogènedécarboxylase,
la coproporphyrinogène oxydase,
la coproporphyrinogène oxydise,
la protoporphyrinogène oxydase
l'uroporphyrinogène III synthase,
la ferrochélatase et
l'uroporphyrinogène décarboxylase,
ou une partie équivalente sur le plan enzymatique ou un analogue de celles-ci.

4. Utilisation selon l'une quelconque des revendications précédentes, où la maladie est la PAI et l'enzyme est la PBGD ou une partie équivalente sur le plan enzymatique ou un analogue de celle-ci.

5. Utilisation selon l'une quelconque des revendications précédentes, où le catalyseur est une forme recombinante de l'enzyme appartenant à la voie de biosynthèse de l'hème ou une partie équivalente sur le plan enzymatique ou un analogue de celle-ci.

6. Utilisation selon l'une quelconque des revendications précédentes, où le catalyseur peut être administré par une voie choisie parmi le groupe constitué par la voie intraveineuse, la voie intraartérielle, la voie intracutanée, la voie souscutanée, la voie orale, la voie buccale, la voie intramusculaire, la voie anale, la voie transdermique, la voie intradermique et la voie intrathècale.

7. Utilisation selon l'une quelconque des revendications 1 à 6, où le catalyseur est formulé dans une solution isotonique, comme une solution de NaCl à 0,9% contenant du phosphate de sodium 10-50 mM, pH 6,50 à 8, ou du phosphate de sodium, de la glycine, du mannitol ou les sels de potassium correspondants.

8. Utilisation selon l'une quelconque des revendications 1 à 7, où le catalyseur est lyophilisé.

9. Utilisation selon l'une quelconque des revendications 1 à 8, où le catalyseur est stérilisé par filtration.

10. Utilisation selon l'une quelconque des revendications 1 à 6, 8 ou 9, où le catalyseur est formulé sous forme de vésicules lipidiques comprenant de la phosphatidylcholine ou de la phosphatidyléthanolamine ou des associations de celles-ci.

11. Utilisation selon l'une quelconque des revendications 1 à 6, 8 ou 9, où le catalyseur est incorporé dans des fantômes érythrocytaires.

12. Utilisation selon l'une quelconque des revendications 1 à 6, 8 ou 9, où le catalyseur est formulé sous forme d'une formulation à libération prolongée comprenant des microsphères biodégradables, comme des microsphères à base d'acide polylactique, d'acide polyglycolique ou de mélanges de ceux-ci.

13. Utilisation selon l'une quelconque des revendications 1 à 9, où le catalyseur est lyophilisé dans une cartouche à deux compartiments, le catalyseur se trouvant dans le compartiment avant et l'eau destinée à la reconstitution dans le compartiment arrière.

14. Utilisation selon la revendication 13 où la cartouche à deux compartiments est associée à un dispositif pour injection destiné à administrer le catalyseur à l'aide d'une aiguille ou d'un dispositif sans aiguilles (sous pression).

15. Utilisation selon l'une quelconque des revendications 1 à 9, où le catalyseur est formulé dans un tampon physiologique contenant un amplificateur pour administration nasale.

16. Utilisation selon l'une quelconque des revendications précédentes, où le catalyseur est formulé sous forme d'une formulation orale contenant des vésicules lipidiques telles que des vésicules à base de phosphatidylcholine, de phosphatidylethanolamine, ou de sphingomyéline ou des microsphères de dextrane.

17. Utilisation selon l'une quelconque des revendications précédentes, où le catalyseur est formulé de manière à prolonger sa demi-vie dans la circulation sanguine du sujet.

18. Utilisation selon la revendication 17, le catalyseur possédant un revêtement de polyéthylèneglycol.

19. Utilisation selon la revendication 17, le catalyseur étant complexé avec un métal lourd.

20. Utilisation selon l'une quelconque des revendications précédentes, où le catalyseur est une partie équivalente sur le plan enzymatique ou un analogue de l'enzyme et exerce au moins une partie de son activité enzymatique de manière intracellulaire après administration au sujet.

21. Utilisation selon la revendication 20, où le catalyseur est une petite enzyme artificielle ou un catalyseur organique qui peut polymériser le porphobilinogène en hydroxyméthylbilane.

22. Utilisation selon l'une quelconque des revendications 1 à 9, où le catalyseur est ladite enzyme formulée de manière à exercer au moins une partie de son activité enzymatique de manière intracellulaire après administration au sujet.

23. Utilisation selon la revendication 22, où le catalyseur est marqué avec des sucres de carbone spécifiques ou d'autres structures spécifiques des cellules hépatiques montrant une absorption hépatique spécifique.

24. Utilisation selon l'une quelconque des revendications 1 à 19, où le catalyseur exerce pratiquement la totalité de son activité enzymatique de manière extracellulaire dans la circulation sanguine.

25. Utilisation selon la revendication 24, où l'activité enzymatique du catalyseur sur le précurseur de l'hème pertinent aboutit à un produit métabolique qui 1) soit passe dans le compartiment intracellulaire et est converti au cours des étapes ultérieures de la voie de biosynthèse de l'hème ou 2) est excrété par le sujet dans l'urine et/ou les fèces.

26. Utilisation selon l'une quelconque des revendications précédentes, où le catalyseur a été préparé à l'aide d'un procédé comprenant les étapes consistant à
a) introduire, dans un vecteur approprié, un fragment d'acide nucléique incluant une séquence d'acide nucléique codant pour le catalyseur ;
b) transformer une cellule hôte compatible avec le vecteur ;
c) cultiver la cellule hôte transformée dans des conditions facilitant l'expression de la séquence d'acide nucléique ; et
d) récupérer le produit d'expression à partir de la culture et éventuellement soumettre le produit d'expression à une maturation post-traductionnelle, comme le repliement de la protéine in vitro, l'élimination enzymatique des partenaires de fusion, l'alkylation des résidus acides aminés et la déglycosylation de manière à obtenir le catalyseur.

27. Utilisation selon l'une quelconque des revendication 1 à 25, où le catalyseur a été préparé par synthèse peptidique en phase liquide ou en phase solide.

28. Utilisation selon l'une quelconque des revendications précédentes, où le catalyseur est exempt de tout autre matériau biologique d'origine humaine.

29. Utilisation selon l'une quelconque des revendications précédentes, où le catalyseur peut être administré au moins une fois par jour, par exemple 2, 3, 4 et 5 fois par jour.

30. Utilisation selon l'une quelconque des revendications précédentes, où la dose quotidienne est de l'ordre de 0,01 à 1,0 mg/kg de poids corporel par jour, comme de l'ordre de 0,05 à 0,5 mg/kg de poids corporel par jour.

31. Utilisation selon l'une quelconque des revendications précédentes, où la dose quotidienne est d'environ 0,1 mg/kg de poids corporel par jour.

32. Utilisation selon l'une quelconque des revendications précédentes, où la composition est adaptée à une administration au moins quotidienne, par exemple répétée 2, 3, 4 et 5 fois par jour et où la dose quotidienne est d'environ 0,1 mg/kg de poids corporel par jour.

33. Utilisation selon l'une quelconque des revendication 1 à 31 où le catalyseur est une forme recombinante de l'enzyme.

34. Utilisation selon l'une quelconque des revendication 1 à 33 où le catalyseur est une PBGD humaine recombinante basée sur l'une quelconque des Seq ID N°1 (clone PBGD 1.1) et Seq. ID N° 12 (PBGD non-érythro 1.1.1).

35. Catalyseur qui est une enzyme de la voie de biosynthèse de l'hème ou une partie équivalente sur le plan enzymatique ou un analogue de celle-ci, destiné à être utilisé en tant que médicament.

36. Catalyseur selon la revendication 35, qui est la PBGD humaine recombinante basée sur l'une quelconque des Seq ID N°1 (clone PBGD 1.1) et Seq. ID N° 12 (PBGD non-érythro 1.1.1).

37. Utilisation d'une séquence d'ADNc de la PBGD humaine de la forme non-érythropoiëtique ou de la forme érythropoiëtique pour la préparation d'un médicament destiné à la thérapie génique par transfection du patient avec la séquence d'ADNc de la PBGD humaine de la forme non-érythropoiëtique ou de la forme érythropoiëtique en fonction du tissu dans lequel la PBGD doit être exprimée, pour la prévention ou le traitement d'une maladie chez un patient portant au sein du gène de la PBGD une mutation responsable d'une carence enzymatique.

38. Utilisation selon la revendication 37, où la carence enzymatique est choisie parmi des carences enzymatiques responsables d'une maladie choisie parmi la porphyrie aiguë intermittente (PAI), la porphyrie à carence en ALA (PDA), la porphyrie cutanée tardive (PCT), la coproporphyrie héréditaire (CPH), l'harderoporphyrie (HDP), la porphyrie variegata (PV), la porphyrie érythropoiëtique congénitale (PEC), la protoporphyrie érythropoiétique (PEP) et la porphyrie hépatoérythropoiëtique (PHP).

39. Utilisation selon la revendication 37, où la maladie est la porphyrie aiguë intermittente (PAI).

40. Utilisation selon l'une quelconque des revendication 37 à 39 où la séquence d'ADNc de la PBGD humaine est choisie parmi les Seq ID N°1 (clone PBGD 1.1) et Seq. ID N° 12 (PBGD non-érythro 1.1.1).

41. Utilisation selon l'une quelconque des revendication 37 à 40 où la transfection est effectuée en utilisant un vecteur choisi entre un adénovirus, un rétrovirus et un adénovirus associé.

42. Utilisation selon l'une quelconque des revendication 37 à 41 où le vecteur de transfert du gène de la PBGD dans les cellules humaines (érythropoiëtiques et/ou non-érythropoiëtiques) aboutit à une activité PBGD normale.

43. Utilisation d'une molécule oligonucléotidique spécifiquement conçue pour la fabrication d'un médicament destiné au traitement par thérapie génique de patients atteints de porphyrie aiguë intermittente (PAI) par correction d'une des mutations ponctuelles spécifiques identifiées responsables de la PAI en utilisant une réparation génique par chiméraplastie.

44. Utilisation selon l'une quelconque des revendication 37 à 43 où le système de délivrance pour la transfection utilise des vecteurs non viraux formulés dans une préparation de support comprenant un ou plusieurs composants choisis parmi des phospholipides cationiques, des phospholipides, des phospholipides mélangés avec des lipides neutres, des PEI lictosylés, des liposomes contenant des mélanges de phospholipides naturels et de lipides neutres.

45. Utilisation selon la revendication 43 ou 44 dans laquelle la mutation est choisie dans le tableau A suivant.
